(19) **Europäisches Patentamt**
**European Patent Office**
**Office européen des brevets**

(11) **EP 2 585 617 B1**

(12) **EUROPEAN PATENT SPECIFICATION**

(45) Date of publication and mention
of the grant of the patent:
**11.05.2016 Bulletin 2016/19**

(51) Int Cl.:
***C12Q 1/68*** (2006.01)

(21) Application number: **11798891.5**

(22) Date of filing: **22.06.2011**

(86) International application number:
**PCT/US2011/041540**

(87) International publication number:
**WO 2011/163425 (29.12.2011 Gazette 2011/52)**

(54) **SEQUENCE SPECIFIC REAL-TIME MONITORING OF LOOP-MEDIATED ISOTHERMAL AMPLIFICATION (LAMP)**

SEQUENZSPEZIFISCHE ECHTZEITÜBERWACHUNG VON LOOP-VERMITTELTER ISOTHERMER AMPLIFIKATION (LAMP)

SURVEILLANCE EN TEMPS RÉEL SPÉCIFIQUE DE SÉQUENCE D'AMPLIFICATION ISOTHERME À MÉDIATION PAR BOUCLE (LAMP)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2010 US 357428 P**

(43) Date of publication of application:
**01.05.2013 Bulletin 2013/18**

(73) Proprietor: **University of Hawaii**
**Honolulu, HI 96822 (US)**

(72) Inventors:
• **KUBOTA, Ryo**
**Honolulu, HI 96822 (US)**
• **JENKINS, Daniel, M.**
**Honolulu, HI 96822 (US)**

(74) Representative: **Lau, Sarah Jane et al**
**Kilburn & Strode LLP**
**20 Red Lion Street**
**London WC1R 4PJ (GB)**

(56) References cited:
**WO-A1-2005/049206      WO-A2-2009/108693**
**US-A1- 2007 275 375      US-A1- 2008 085 515**
**US-A1- 2010 055 685**

• **NOTOMI T ET AL: "LOOP-MEDIATED ISOTHERMAL AMPLIFICATION OF DNA", NUCLEIC ACIDS RESEARCH, INFORMATION RETRIEVAL LTD, vol. 28, no. 12, 1 June 2000 (2000-06-01), pages I-VII, XP002907443, ISSN: 0305-1048, DOI: 10.1093/NAR/28.12.E63**
• **YI ET AL.: 'Molecular Zipper: a fluorescent probe for real-time isothermal DNA amplification.' NUCLEIC ACIDS RESEARCH vol. 34, no. 11, 2006, pages 1 - 5, XP002434420**
• **KUBOTA ET AL.: 'Detection of Ralstonia solanacearum by Loop-Mediated Isothermal Amplification.' BACTERIOLOGY vol. 98, no. 9, 2008, pages 1045 - 1051, XP055073940**
• **JUSSI NURMI ET AL: "High-Performance Real-Time Quantitative RT-PCR Using Lanthanide Probes and a Dual-Temperature Hybridization Assay", ANALITICAL CHEMISTRY, AMERICAN CHEMICAL SOCIETY, vol. 74, no. 14, 15 June 2002 (2002-06-15) , pages 3525-3532, XP007919499, DOI: 10.1021/AC020093 [retrieved on 2002-06-17]**

**Description**

CROSS-REFERENCE TO RELATED APPLICATIONS

This application claims priority to U.S Provisional Patent Application No. 61/357,428, entitled "SYSTEMS AND METHODS FOR SEQUENCE SPECIFIC REAL-TIME MONITORING OF LOOP-MEDIATED ISOTHERMAL AMPLIFICATION (LAMP)", filed June 22, 2010.

STATEMENT REGARDING FEDERALLY SPONSORED R&D

This invention was made with Government support under Grant Nos. 2006-55605-16683 and 07-55605-17843 and project HAW00559-04G awarded by the United States Department of Agriculture (USDA-NRI). The Government has certain rights in this invention.

BACKGROUND

Pathogenic bacteria are those bacteria that can cause disease in plants, animals, and human beings. Owing to the diversity of pathogenic bacteria, a large variety of pathogenic bacteria and attendant diseases exist. Examples of these diseases may include wilt, soft rots, and blight in plants, parvovirus, giardia, and Rocky Mountain Spotted Fever in dogs, and tuberculosis, pneumonia, and tetanus in human beings.
The diseases caused by bacterial pathogens may cause significant damage to property and life. For example, *Ralstonia solanacearum* is a bacterial pathogen that causes wilt in over 200 plant species. One species of this pathogen that primarily affects potato crops, race 3 biovar 2, has been estimated to cause damages in excess of about $950 million each year.
Slowing and preventing the transmission of bacterial diseases first involves identifying the presence of the pathogenic bacteria. However, techniques for detection of pathogenic bacteria may be relatively slow. Furthermore, these techniques may be unable to discriminate selected species of a bacterial pathogen from other species. As a result of the inability to properly identify biological pathogens, actions to contain and eradicate outbreaks may be unduly delayed.
Therefore, there is significant interest in developing detection technologies that are capable of detecting selected pathogen strains rapidly and discriminating sub-populations of the selected pathogen strain from other populations within the same species.

SUMMARY

In one aspect, the present invention provides a method of monitoring LOOP-mediated isothermal amplification (LAMP) of a target DNA, comprising:

contacting an assimilating probe and a DNA polymerase with a LAMP reaction mixture,
wherein the LAMP reaction mixture comprises the target DNA and one or more LAMP primers that hybridize with the target DNA,
wherein the assimilating probe comprises a first and second oligonucleotide strand, wherein the first oligonucleotide strand comprises a quencher probe at a 3' end and wherein the second oligonucleotide strand of the assimilating probe comprises a fluorophore at a 5' end;
wherein the ratio of the amount of the second oligonucleotide strand to the amount of the first oligonucleotide strand is less than 1:1; and
measuring fluorescence emitted by the LAMP reaction mixture that has been contacted with the assimilating probe and the DNA polymerase.

In another aspect, the present invention provides the use of a composition comprising an assimilating probe for monitoring LOOP-mediated isothermal amplification (LAMP) of a target DNA, wherein the composition comprises:

a first oligonucleotide strand comprising a quencher probe at a 3' end of the first oligonucleotide strand; and
a second oligonucleotide strand comprising a fluorophore at a 5' end of the second oligonucleotide strand;
wherein the ratio of the amount of the second oligonucleotide strand to the first oligonucleotide strand is less than 1:1.

In another aspect, the present invention provides a method of detecting the presence or absence of a target DNA comprising:

contacting an assimilating probe and a DNA polymerase with a LAMP reaction mixture,

wherein the LAMP reaction mixture comprises a sample to be tested for the presence of a target DNA and one or more LAMP primers capable of amplifying the target DNA,

wherein the assimilating probe comprises a first and second oligonucleotide strand, wherein the first oligonucleotide strand comprises a quencher probe at a 3' end and wherein the second oligonucleotide strand of the assimilating probe comprises a fluorophore at a 5' end;

wherein the ratio of the amount of the second oligonucleotide strand to the amount of the first oligonucleotide strand is less than 1:1; and

detecting the presence or absence of the target DNA.

BRIEF DESCRIPTION OF THE DRAWINGS

Figure 1 is a schematic illustration of a circuit for a laser driver for temperature control and fluorometer for monitoring hybridization probes;

Figure 2 is a schematic illustration of radiation sources in the CD-based platform;

Figure 3A is a schematic illustration of a circuit board layout for a custom fluorometer and laser driver of an embodiment of the present disclosure;

Figure 3B is an optical photograph of a populated circuit board for the custom fluorometer and laser driver of Figure 3A. The pyrometer head is further illustrated above a polycarbonate CD;

Figure 4 is an optical photograph of a printed CD in ABS with various size reaction wells;

Figure 5 is a plot of corrected well temperatures estimated from a non-contact thermometer (e.g., pyrometer) as compared to actual well temperatures measured by a thermocouple. Calibration data used to estimate coefficients for transmission of radiation through well and reflection of ambient radiation from well surface are illustrated in the inset;

Figure 6 is a plot of temperature measurements showing temperature control in a reaction well from about ambient temperature to a set point of about 65°C;

Figure 7 is a plot of relative fluorescence versus time for rsfliC LAMP with about 0.4 $\mu$M of a molecular zipper;

Figure 8 is a plot of relative fluorescence versus time illustrating real-time monitoring of rsfliC LAMP reaction by addition of fluorescence strand and quencher strand directly in the LAMP reaction mix prior to formation of the molecular zipper structure;

Figure 9 is a plot of relative fluorescence versus time for rsfliC LAMP and lambda phage LAMP with rsfliC targeted assimilating probe (0.08 $\mu$M of fluorescence strand and 0.16 $\mu$M of quencher strand);

Figure 10 is a plot of relative fluorescence versus time illustrating the use of molecular beacons for end point detection of rsfliC LAMP amplications and speed observations of rsfliC LAMP reaction;

Figure 11 is a plot of relative fluorescence versus time illustrating measured fluorescence when using the rk2208.1 and rsfliC primer sets in combination with increased concentrations of *Bst* DNA polymerase;

Figure 12 is a plot of relative fluorescence versus time illustrating observable fluorescence increases obtained using the rk2208.1 primer set with increased concentrations of *Bst* DNA polymerase;

Figure 13 is a plot of time to reach a detection threshold vs. *Bst* DNA polymerase content in data from Figure 12;

Figure 14 is a plot of relative fluorescence versus time for LAMP reactions with primer set rk2208.1, without the loopF primer, that is monitored with an assimilating probe. The fluorescence strand was provided in concentrations of about 0.08 $\mu$M, about 0.4 $\mu$M, and about 0.8 $\mu$M and quencher strand was provided in concentrations of about 0.16 $\mu$M, about 0.8 $\mu$M, and about 1.6 $\mu$M ;

Figure 15 is a plot of relative fluorescence versus time for rk2208.1 LAMP reactions with loopF primer with different concentrations of assimilating probe fluorescence strands and quencher strands. The concentration of the quencher strands was approximately twice that of the fluorescence strands;

Figure 16 is a plot of relative fluorescence versus time for LAMP reactions using embodiments of the assimilating probe of the present disclosure and intercalating dye (EvaGreen);

Figure 17 is a plot of relative fluorescence versus time for multiplexed LAMP reactions for *Salmonella enterica* and bacteriophage lambda genomic DNAs;

Figure 18 is a plot of relative fluorescence observed using spectral settings for fluorescein versus time for multiplexed LAMP reactions amplifying *Salmonella enterica* and/or bacteriophage lambda genomic DNAs using fluorescein labeled assimilating probes for *Salmonella enterica;* and

Figure 19 is a plot of relative fluorescence observed using spectral settings for cy3 versus time for multiplexed LAMP reactions amplifying *Salmonella enterica* and bacteriophage lambda genomic DNAs using cy3 labeled assimilating probes for lambda phage.

DETAILED DESCRIPTION

The terms "approximately," "about," and "substantially" as used herein represent an amount close to the stated amount that still performs a desired function or achieves a desired result. For example, the terms "approximately," "about," and "substantially" may refer to an amount that is within less than 10% of, within less than 5% of, within less than 1% of, within less than 0.1% of, or within less than 0.01% of the stated amount.

Embodiments of the disclosure present gene-based diagnostics capable of rapidly discriminating selected strains of selected pathogens from other populations within the same species. Examples may include, but are not limited to, bacteriophage lambda, race 3 biovar 2 strains of *Ralstonia solanacearum, Ralstonia solanacearum, Salmonella enterica,* and *Staphylococcus aureus.* Sequence-specific, real-time monitoring of LOOP-mediated isothermal amplification (LAMP) of target DNA may be accomplished through the addition of particular oligonucleotide probes, referred to herein as "assimilating probes," to a mixture including LAMP primers and the target DNA.

The assimilating probes themselves include two distinct oligonucleotide strands. A first oligonucleotide strand includes a quencher (referred to as the quenching probe) and a second oligonucleotide strand includes a fluorophore (referred to as the fluorescent probe). A fluorescent signal results when the two strands are displaced from one another during the LAMP reaction. By monitoring the emitted fluorescence, the LAMP reaction may be detected.

Furthermore, embodiments of the assimilating probes may include strands that undergo substantial displacement when amplification of a selected target DNA occurs. Therefore, the assimilating probes may not substantially fluoresce when amplification of the target DNA does not occur and may substantially fluoresce when amplification of the target DNA is present. Thus, detection of amplification of the target DNA may be sequence-specific.

As used herein, the term assimilating probe may adopt its customary meaning as understood by one of skill in the art, and may further refer to the fluorescent probe and the quenching probe when not hybridized with each other and also when the fluorescent probe and the quenching probe are hybridized together.

As discussed in greater detail below, the presence of the assimilating probes within the LAMP reaction mixture may slow the rate at which the LAMP reaction proceeds. However, parameters of the assimilating probes have been identified that reduce the effect of the assimilating probe on the LAMP reaction rate due to the presence of the assimilating probe. These parameters include, but are not limited to, the ratio of the fluorescent probe to the quencher probe, the manner of mixing the strands (the fluorescent probe and the quencher probe) to create the assimilating probe, and the total quantity of the assimilating probe. By reducing the effects of the assimilating probe on the LAMP reaction rate, real time monitoring of the LAMP reaction is facilitated. These parameters may also be varied within selected ranges to reduce the time required to detect the LAMP reactions, further facilitating real-time detection.

Embodiments of assimilating probes designed for separate LAMP reactions can also be used for real-time detection of more than one unique gene sequences (multiplexed detection). In order to perform multiplexed detection of LAMP reactions in real time, each assimilating probe may employ spectrally unique fluorophore that can be monitored independently.

Further embodiments of the disclosure present a substrate-based hardware platform that facilitates application of these gene-based diagnostics in real-time. In some embodiments, the platform includes a compact disc (CD) or other substrate patterned with wells for performing LAMP reactions. In some embodiments, a pyrometer may be employed as a non-contact temperature sensor to monitor the temperature within reaction wells of the substrate-based platform. The pyrometer may estimate the temperature within the reaction wells of the substrate based upon measurements of thermal radiation emitted from the reaction well. A calibration procedure is further developed to correct errors in the temperature measured by the pyrometer due to the presence of water and a transparent film that seals the reaction wells. These temperature measurements may be further used to control a heating source. In this manner, the temperature within the reaction wells may be kept approximately constant for the LAMP reactions occurring therein.

Beneficially these tools may facilitate accurate discrimination of pathogens and, for example, enable timely management decisions to be made in response to introduction of the disease. It may be understood that the disclosed embodiments may be useful in many other contexts, as well, including, but not limited to, clinical diagnostics, such as in low resource settings, and identification of biological agents by military and homeland security personnel.

In an embodiment, a method of real time detection of selected sequences of DNA by LAMP is provided. The method may comprise forming patterns in a selected substrate (e.g., a CD) to form reaction wells. The method may further comprise adding a LAMP reaction mixture and an assimilating probe, as described herein, to the reaction wells. The method may additionally comprise sealing the wells and adding a target DNA to the wells. The method may additionally comprise heating the contents of the wells with a heat source (e.g., a laser) to a temperature sufficient to induce fluorescence. The method may also comprise detecting the fluorescence.

**Hardware Design**

The LAMP processes may be carried out on a substrate comprising a plurality of wells for individual LAMP reactions. The substrate may be of any material known in the art, such as plastics, metals, or composites. In some embodiments, a compact disc (CD) platform may be used to conduct Loop Mediated Isothermal Amplification (LAMP) processes for DNA amplification and detection. The CD platform allows for automation of sample preparation and reaction steps, as well as provides good containment. Although generally described herein in terms of a CD substrate, other substrates that provide a plurality of reaction wells may be used.

LAMP reactions are performed within reaction wells contained within the CD or other substrate. In certain embodiments, the reaction wells may be fabricated by patterning. Patterns in the substrate, such as CDs, may be created by mechanisms including, but not limited to, lasers and/or printing for rapid prototyping, injection molding for volume production, and other mechanisms for patterning compact discs and other substrates as known in the art.

For example, in one embodiment, patterns may be created using a laser engraver (e.g., Venus-30, GCC Systems, Taipei Hsien, Taiwan) on discs of black polycarbonate (e.g., Lexan). In another embodiment, polycarbonate blank discs may be patterned with a computer numerical controlled (CNC) machine. In a further embodiment, patterned acrylonitrile butadiene styrene (ABS) discs may be produced in their entirety using a 3D printer (e.g., SST 1200, Dimension Inc, Eden Prairie MN). The cross-sectional shape of the discs may be fabricated in accordance with the desired application. For example, in certain embodiments, the wells may be fabricated in a cylindrical configuration having a generally circular cross-section.

In some embodiments, the thickness of the substrates employed for LAMP monitoring may be within the range between about 1.2 mm to about 1.8 mm. It may be understood, however, that embodiments of the substrates may be formed from materials such as other polymers as known in the art, without limit.

The cross-sectional area of the reaction wells may also be varied, as necessary. For example, in embodiments of wells having cylindrical configurations with a generally circular cross-section, the diameter of the reaction well may be varied based upon the particular reaction performed in the well, without limit. In some embodiments, the value of the well diameter may be from about 3 mm to about 4 mm, or any value in between.

In further non-limiting examples, one or more of the reaction wells may be surrounded by one or more of a wall and a channel. Beneficially, the presence of the wall and/or channel may improve the insulation around a reaction well. The value of the wall thickness, in certain embodiments, may be selected within the range between about 0.5 mm to 5 mm. The value of the channel thickness, in certain embodiments, may be selected to have a value within the range from about 0.5 to about 5 mm. It may be understood, however, that the wall thickness and channel thickness may be selected to adopt any value suitable for LAMP monitoring, as necessary. Unless otherwise noted, the reaction well thickness and channel thickness employed in the examples discussed below were, respectively, about 1 mm and about 2 mm. In some embodiments, the depth of the reaction well and channel may be selected to retain 5mm, 4mm, 3mm, 2mm, 1mm, 0.5mm, 0.3 mm or less of substrate material under the reaction well and/or channel. In the examples below, the depth of the reaction well and channel was selected to retain about 0.3 mm of the thickness of a disc material under the reaction well.

The temperature of the CD or other substrate can be recorded using a non-contacting temperature sensor. In one embodiment, the non-contacting temperature sensor may comprise a pyrometer that includes a light source and a detector. For example, a commercial pyrometer with a miniature optical head may be attached to a flexible cable (e.g., MID20LTCB3, Raytek, Santa Cruz, CA). Alternatively, a monolithic type (e.g. MLX90614ESF-BAA, Melexis, Ieper Belgium) may be mounted directly on a circuit board adjacent to the placement of the reaction well. For calibration, the standard temperature may be recorded using a thermocouple (e.g., type K thermocouple made from fine thermocouple wire, e.g., Omega Engineering, Stamford, CT). The thermocouple wire may be connected to a software compensated multi-meter (e.g., Fluke 186, Everett, WA).

As described in greater detail below, in order to facilitate calibration of the non-contacting temperature sensor, the wells may be substantially filled with distilled water (or other non-reactive liquid) and covered with a transparent film with adhesive backing (e.g., Microseal 'B' adhesive seals, Bio-Rad, Hercules CA). Beneficially, by calibrating the non-contacting temperature sensor to compensate for effects of non-emissive liquids and films in the reaction well,, the temperature within the reaction wells may be more precisely controlled.

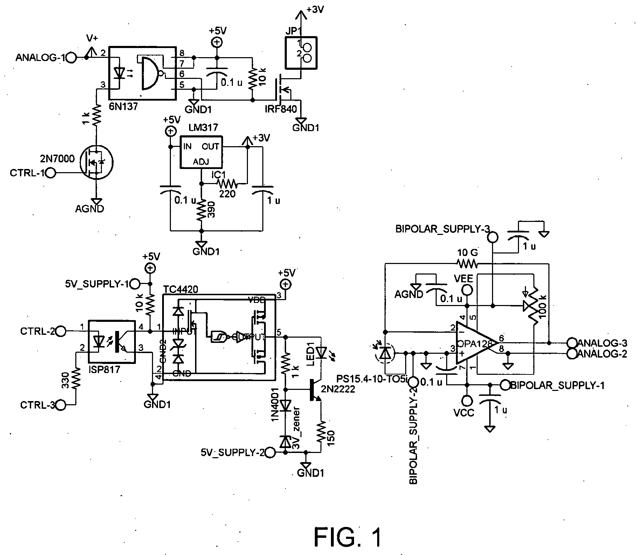

Heating of the reaction wells may also be performed by heat source. Heat sources may include, but are not limited to, contacting and non-contacting sources, as known in the art. In one embodiment, the heat source may comprise an optical heating device. For example, the optical device may comprise a defocused laser that is directed at an underside of the reaction wells. For example, heating may be achieved by using an 808 nm infra-red laser diode module (e.g., icetec-UK) operating at approximately 150 mW directed approximately onto the underside of the well. The laser may be approximately defocused to inhibit burning of the disc and to distribute heat evenly in the well. The power of the laser may be controlled through an n-channel power MOSFET gated by a logic optocoupler (see Figure 1) driven by pulse width modulated (PWM) signal from a microcontroller (e.g., Fox LP3500, Rabbit Semiconductor, Davis, CA).

To provide temperature control, the controller may be programmed with a modified proportional-integral control routine using feedback from the pyrometer. The pyrometer feedback may be received by the microcontroller after a calibration correction is applied, as described below. To perform optical temperature detection, the sample may be illuminated obliquely, for example, by a high intensity light source having a selected wavelength. In one embodiment, the light source may comprise a blue light emitting diode (LED) that emits light at a wavelength selected within the range between about 450 nm to about 475 nm (e.g., approximately 470 nm). An example of an LED light source capable of this illumination is HLMP CB28 STD00, manufactured by Agilent Technologies, Santa Clara, CA.

Thermal radiation emitted from the reaction well may be detected by a detector. In an embodiment, the detector may comprise a photodiode positioned adjacent the light source. The detector may be mounted to collect thermal radiation from the surface of the well that has traveled through the water and the transparent film. The detector may be operated using a high gain photoamplifier circuit (e.g., T-5 Series, Intor Inc., Socorro NM) operating within the range between about $10^{10}$ V/A - $10^{12}$ V/A, as illustrated in Figure 1. The excitation and emission spectra measured by the detector may be tuned using narrow band pass interference filters (e.g., Intor). It may be understood, however, that in alternative embodiments, a contact temperature sensor may be used in lieu of or in conjunction with the above-described photodiode detector.

**Calibration of Non-Contact Temperature Sensor**

A non-contact temperature sensor may be selected for temperature measurement in the system so as to prevent contamination, such as in the disposable CD platform. For example, a pyrometer may be employed as the non-contact temperature sensor. However, the detector measures thermal radiation emitted from the surface of the reaction well in order to determine the temperature in the area probed (e.g., a temperature of a an individual well or average temperature of a selected group of wells). This thermal radiation passes through the reaction liquid (e.g., the water) and the material employed to seal the wells (e.g., the transparent film) the presence of film). Without being limited by theory, it is believed that, as these materials possess low emissivity, they may introduce reflections in the radiation that originates from inside the well as well as the thermal radiation from the surroundings. As a result, these materials may introduce errors in the temperature calculated from the thermal radiation measured by the pyrometer.

The pyrometer may be calibrated to correct for these errors. As discussed below, the temperature within a reaction well, as a function of different optical powers from the heating source in the steady state, may be measured using the pyrometer, as well as thermocouples in contact with the reaction well. These measurements may be input into a calibration model that calculates the correct temperature of a reaction well based upon the temperature measured by the pyrometer.

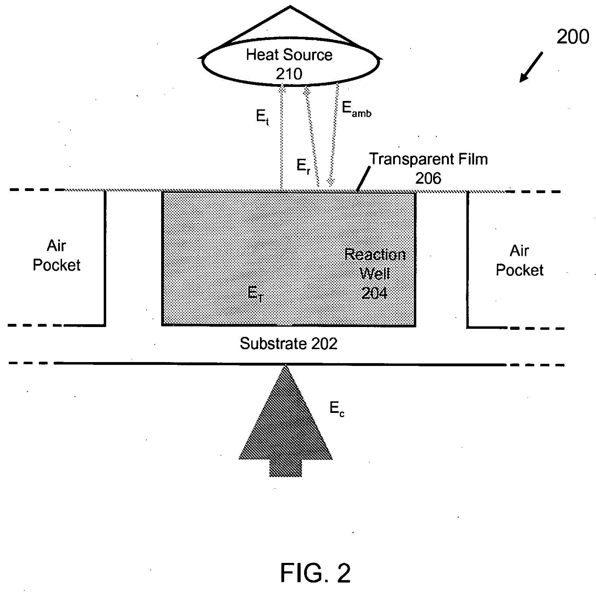

A CD-based detection system 200 is schematically illustrated in Figure 2 along with radiation sources. The system 200 includes a substrate 202 (e.g., a plastic disk such as polycarbonate, Acrylonitrile Butadiene Styrene (ABS), or another suitable plastic material) having one or more reaction wells 204. A transparent film 206 may be placed over the reaction well 204 to inhibit contaminants from entering the reaction wells 204.

A detector 210 may be positioned above the substrate 202 to measure thermal radiation emitted from the substrate 202. In some embodiments, the detector 210 may comprise a pyrometer. A calibration model may be employed to correct errors in the temperature measurements of the detector 210 due to transmission of the thermal radiation through materials within the reaction well (e.g., the LAMP reaction mixture, protective film covering the well) and absorbance of incident fluorescence by the substrate itself. As illustrated in Figure 1, $E_c$ is the thermal radiation that is absorbed into the substrate 202 from the heat source to control the temperature of the reaction well 204. $E_T$ is the thermal radiation emitted from the surface of the reaction well 204. $E_t$ is the portion of thermal radiation from the surface of the reaction well 204 that is transmitted through the reaction well 204 and the transparent film 206 to reach the detector 210. $E_{amb}$ is the thermal radiation emitted from the surroundings that impinges on the transparent film 206. $E_r$ is the portion of the ambient thermal energy that is reflected into the detector 210. In an embodiment, the calibration model may assume that substantially all of the radiation that originates from the surface of the reaction well 204 or from the surroundings and is detected at the detector 210 after crossing either through the water in the reaction well 204 and the transparent film 206 or after reflecting off of these surfaces.

The solutions of Maxwell's equations under these circumstances result in reflection and transmission coefficients that are related to the normalized wave impedances of the various materials at their respective boundaries. These wave impedances are, in turn, related to the respective electric permittivities and magnetic permeabilities of the materials through which the wave passes. The net result is that a fraction, a, of the total thermal radiation originating from the reaction well 204 reaches the detector 210 and a fraction, b, of the total thermal radiation originating from the surroundings is reflected away from the reaction well 204 to reach the detector 210.

From Stefan-Boltzmann's law applied to a gray body, the total thermal energy emitted by a body, E, is related to the emissivity, $\varepsilon$, of the material and the absolute temperature T of the surface of the transparent film 206 according to Equation 1:

$$E = \varepsilon \sigma T^4 \qquad (1)$$

where $\sigma$ is the Stefan-Boltzmann constant. The detector 210 may estimate the temperature of the surface of the transparent film 206 based upon the thermal radiation reaching the detector of the pyrometer 210, assuming a selected emissivity of the material. The total thermal energy that reaches the detector of the pyrometer 210, E, may be used to calculate the indicated temperature $T_i$ according to Equation 2:

$$E_i = E_t + E_r \qquad (2)$$

$E_t$ is the thermal radiation originating from the reaction well 204 that reaches the detector pyrometer 210 after transmission through the volume of reaction material within the reaction well 204 and the transparent film 206. $E_r$ is the thermal energy from the surroundings that reaches the detector of the detector 210 after reflecting off of the surface of the transparent film 206 (see, e.g., Figure 2).

Applying Stefan-Boltzmann's law, and empirical coefficients a and b from above to each of the terms in the preceding equation, the Equation 3 may be obtained:

$$\varepsilon_{set} \sigma T_i^4 = a \varepsilon_{well} \sigma T^4 + b \varepsilon_{amb} \sigma T_{amb}^4 \qquad (3)$$

$\varepsilon_{Set}$ is the emissivity setting of the detector 210. In certain embodiments, a default value may be assumed for $\varepsilon_{set}$ (e.g., about 0.95). However, other emissivity values may be adopted, depending upon the detector 210. $\varepsilon_{well}$ is the emissivity of the reaction well surface. $\varepsilon_{amb}$ is the emissivity of the surrounding materials at ambient temperature $T_{amb}$.

The temperatures $T_i$, $T_{amb}$, and T may be related to each other. For example, by rearranging Equation 3, $T_i$ may be expressed in terms of $T_{amb}$, T, and empirical coefficients, referred to as A and B, and the ambient environment, including corrections for transmission and/or reflection losses:

$$T_i^4 = A T^4 + B T_{amb}^4 \qquad (4a)$$

$$A = (a\varepsilon_{well})/(\varepsilon_{set}) \qquad (4b)$$

$$B = (b\varepsilon_{abl})/(\varepsilon_{set}) \qquad (4c)$$

The empirical coefficients A and B represent the ratios of the emissivities of the system (e.g., the well) and may be determined by performing a linear regression of the fourth power of the indicated temperatures $T_i$ as a function of the fourth power of the true well temperatures T, at a constant ambient temperature $T_{amb}$. Solving Equation 4a for the actual well temperature T yields coefficients A and B. These coefficients, in turn, may used to estimate T from a given detector temperature reading $T_i$ according to Equation 5:

$$T = \left( \frac{T_i^4 - B T_{amb}^4}{A} \right)^{1/4} \qquad (5)$$

In this manner, the detector 210 can be calibrated to improve the accuracy of the non-contact temperature measurements. However, in other embodiments, the temperature measurements may be obtained by other mechanisms, such as by a thermocouple in contact with the reaction well.

**Fluorometer and Laser Driver**

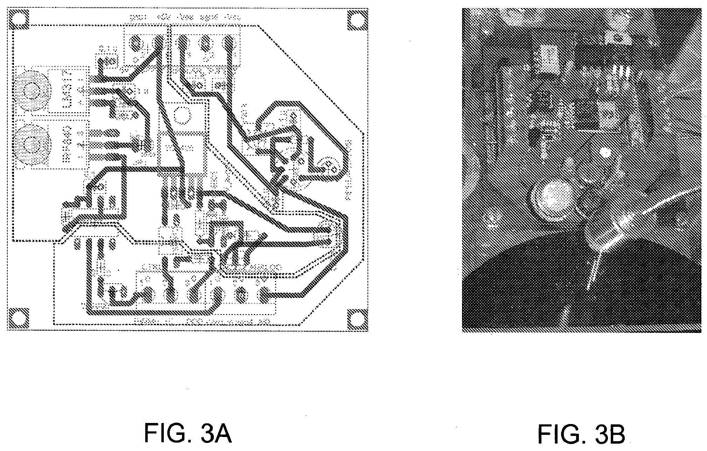

Figure 3A is a schematic illustration of a circuit board layout for a custom fluorometer and laser driver of an embodiment of the present disclosure.

Figure 3B is a photograph of a populated circuit board for the custom fluorometer and laser driver of Figure 3A. A pyrometer head is further illustrated above a polycarbonate CD.

**Loop Mediated Isothermal Amplification and Detection:**

Loop-mediated amplification (LAMP) can be used to achieve gene replication isothermally and without denaturation of the template DNA. LAMP also can amplify its target DNA without lysing or extracting cells but which results in lower detection limit. Embodiments of LAMP are described in detail in the following documents.

- N. Tsugunori, et al., "Loop-mediated isothermal amplification of DNA," Nucleic Acids Research, Vol. 28, No. 12, June 15, 2000.
- Y. Mori, et al., "Detection of loop-mediated isothermal amplification reaction by turbidity derived from magnesium pyrophosphate formation," Biochemical. Biophys. Res. Comm., Vol. 289, No. 1, p. 150-154, Nov. 23, 2001.
- K. Nagamine, "Isolation of single-stranded DNA from loop-mediated isothermal amplification products," Biochemical and Biophysical Research Communications, Vol. 290, No. 4, p. 1195-1198, Feb 1, 2002.
- K. Nagamine, et al., Accelerated reaction by loop-mediated isothermal amplification using loop primers," Molecular and Cellular Probes, Vol. 16, No. 3, p. 223-229, June 2002.

In an embodiment, LAMP may employ a set of four primers. These four primers may recognize six distinct sequences and rely on auto-cycling strand-displacing DNA synthesis, for example by the *Bst* DNA polymerase large fragment. The primers for the LAMP reaction may be the inner primers (e.g., FIP and BIP) and the outer primers (e.g., F3 and B3). The LAMP reaction may be initiated by hybridization of either inner primer (e.g., FIP or BIP) to its respective priming site (e.g., F2c or B2c) on the target DNA. The outer primer (e.g., F3 or B3) secondarily hybridizes to its priming site (e.g., F3c or B3c) on the target DNA and initiates synthesis of a new complementary sequence that displaces DNA sequences already extended from the inner primer. The result is a DNA sequence which can form stem-loop structures at both ends. This autoprimed "dumb-bell" structure is the starting material for LAMP auto-cycling amplification.

The LAMP reaction can also be accelerated using additional primers, referred to as loop primers. The loop primers may hybridize to sections of the loop which were transcribed from the target DNA template. This additional priming may accelerate the LAMP reaction and improve LAMP selectivity as it requires transcription of the correct starting material. In certain embodiments, the LAMP reaction may be conducted under isothermal conditions at temperature values selected within the desired range, for example between about 60°C to about 70°C, or about 60°C to about 65°C. The amplification products may be stem-loop DNAs, which typically possess several inverted repeats of the target. The inverted repeats typically exhibit a cauliflower-like structure with multiple loops.

A variety of mechanisms have been developed to detect positive amplification of DNA by LAMP. In one aspect, positive amplification of DNA may be monitored in real-time using intercalating dyes such as SYBR Green or EvaGreen®. SYBR Green is known to have an inhibitory effect for Polymerase Chain Reaction (PCR) or thermophilic helicase-dependent amplification (tHDA). In contrast, EvaGreen has been reported to have a lower inhibitory effect for PCR and also been used for detect the LAMP reaction. However, as discussed in greater detail below, high inhibitory effect of EvaGreen for LAMP reactions may be observed. Furthermore, this mechanism does not allow sequence specific confirmation of the DNA product. Notably, however, this mechanism does not allow sequence specific confirmation of the DNA product. Rather, the technique merely detects positive amplification.

In another embodiment, a positive LAMP reaction can be identified by measuring white turbidity of the LAMP reaction mixture. The white turbidity is indicative of a positive LAMP reaction, as magnesium pyrophosphate is a by-product of the LAMP reaction. However, while turbidity may provide an indication that positive amplification occurs, the turbidity itself is not a conclusive indicator that a specific sequence of interest has undergone LAMP. Therefore, reliance of turbidity alone to determine sequence specificity is susceptible to higher rate of false positives.

Fluorescence resonance energy transfer (FRET) based detection methods such as molecular beacons, Taqman probes, and molecular zippers are techniques that provide for real-time, sequence-specific monitoring of DNA amplification processes. A molecular beacon comprises a nucleic acid probe that possesses a self-complementary stem-loop structure that is conjugated to a fluorescent molecule at one end and a quencher molecule at the opposite end. In the absence of target sequence, no fluorescence is emitted, as the quencher is close to the fluorophore because of complement stem loop sequence. When the loop region hybridizes to the target, the quencher and fluorophore are separated from each other and fluorescent emission can be detected.

Notably, however, the use of molecular beacons for monitoring LAMP can be difficult. For example, molecular beacons may be poorly accessible to dsDNA products such as LAMP amplicons under isothermal conditions. Furthermore, Taqman probes are generally not employed to detect LAMP reactions as *Bst* DNA polymerase lacks exonuclease activity. Nevertheless, any of these techniques may be employed in certain embodiments.

Molecular zippers are molecules comprising a partially double stranded short DNA fragment. A first strand of the molecular zipper includes a quencher at the 3' end and may be referred to as a quenching probe. A second strand of the molecular zipper includes a partially complementary strand having a fluorophore conjugated on the 5' end and a priming sequence complementary to a selected bit of target DNA such as lambda phage F, lambda phage B, rk1249.1 F, rk2208.1 F, rk2403.1 F, rsfliC B, rsfliC F, Se01 F, SE01 B, spa1.. This second strand may be referred to as a fluorescent probe. Examples of the quencher may include, but are not limited to, DABCYL, TAMRA, and the Black Hole Quenchers (BHQ) (Biosearch Technologies, Novato, CA). Examples of the fluorophore may include, but are not limited to, fluorescein, cy3, cy5, and any number of quantum dots as known in the art. The fluorophore conjugated end aligns with the quencher in the assembled zipper construct. In certain embodiments, the strands of the molecular zipper do need to be complementary, with the exception of the overhanging unmatched segment of the fluorescent strand which acts as a primer in a DNA polymerization reaction.. In some embodiments, the molecular zipper may be configured such that, when the two strands of the zipper are hybridized to each other, fluorescence is substantially quenched.

A fluorescent signal results from the molecular zipper when the two strands of the zipper are displaced. As a result, molecular zippers can be used in LAMP. In further embodiments, by designing the overhanging region to hybridize to the loop region of the LAMP product, separation of the molecular zipper by the strand-displacing activity of *Bst* DNA polymerase may occur during the course of the ensuing polymerization reaction. Also, in further embodiments, by configuring the zipper sequence with a melting temperature in excess of that of the LAMP process (e.g., about 65°C), molecular zippers can be used for real-time monitoring in LAMP reaction with low background signal. Molecular zippers have been previously been demonstrated for real-time monitoring of Rolling-circle amplification (RCA) and ramification amplification (RAM).

In molecular zippers, the fluorescent probe and the quencher probe are typically mixed in approximately equal amounts and hybridized to form double stranded zippers. For example, in certain embodiments, equal amounts of the fluorescent probe and quencher probe (e.g., about 20 $\mu$M) may be incubated at about 95°C for about 5 min, and cooled slowly to about room temperature in order to form the double-stranded molecular zipper.

The hybridized molecular zipper probe is added to the LAMP reaction mixture. In some embodiments, the hybridized molecular zipper probe may be added to provide a final concentration value selected in the range between about 0 to about 100 $\mu$M. In certain embodiments, the concentration of the molecular zipper may have a value selected within the range between about 0 $\mu$M to about 10 $\mu$M. In additional embodiment, the concentration of the molecular zipper may be within the range between about 0 $\mu$M to about 1 $\mu$M. In one embodiment, the concentration of the molecular zipper may be about 0.4 $\mu$M.

In some embodiments, the present disclosure provides improved detection techniques. A pair of labeled oligonucleotide probes, herein referred to as an "assimilating probe" has been designed to perform sequence-specific, real-time monitoring LAMP of DNA using the principle of FRET. The probe pair is analogous to the molecular zippers discussed above. However, the molecular zippers and embodiments of the assimilating probes of the present disclosure are at least different with respect to their implementation of the reaction process.

Table 1 illustrates embodiments of fluorescent probe and quencher probe for use with different LAMP primers and target organisms.

**Table 1 - LAMP Primer Sets and Assimilating Probes for Target Organisms**

| LAMP Primer Set | Target Organism | Assimilating Probe F Strand | Assimilating Probe Q Strand | Reaction Temp (°C) |
|---|---|---|---|---|
| Lambda phage LAMP | Bacteriophage lambda | Lambda phage F probe | Quencher probe 01 | 65 |
| | | Lambda phage B probe | | |
| rk1249.1 | *Ralstonia solanacearum* Race3 Biovar2 | rk1249.1 F probe | Quencher probe 02 | 65 |
| rk2208.1 | *Ralstonia solanacearum* Race3 Biovar2 | rk2208.1 F probe | Quencher probe 01 | 65 |
| rk2403.1 | *Ralstonia solanacearum* Race3 Biovar2 | rk2403.1 F probe | Quencher probe 01 | 67 |
| rsfliC LAMP | *Ralstonia solanacearum* | rsfliC F probe | Quencher probe 01 | 65 |
| | | rsfliC B probe | | |
| Se01LAMP | *Salmonella enterica* | Se01 F probe | Quencher probe 01 | 65 |
| | | Se01 B probe | | |

(continued)

| LAMP Primer Set | Target Organism | Assimilating Probe F Strand | Assimilating Probe Q Strand | Reaction Temp (°C) |
|---|---|---|---|---|
| SpaILAMP | *Staphylococcus aureus* | spa1 B probe | Quencher probe 01 | 65 |

The molecular structure/sequence of each of primers and probes are summarized in Table 2.

| LAMP Primer Set | Target Organism | Assimilating Probe F Strand | Assimilating Probe Q Strand |
|---|---|---|---|

Table 2 - LAMP primers, assimilating probes, and molecular beacons

| LAMP primer sets | | |
|---|---|---|
| | lambda phage | |
| | F3 | GGCTTGGCTCTGCTAACACGTT |
| | B3 | GGACGTTTGTAATGTCCGCTCC |
| | FIP | CAGCCAGCCGCAGCACGTTCGCTCATAGGAGATATGGTAGAGCCGC |
| | BIP | GAGAGAATTTGTACCACCTCCCACCGGGCACATAGCAGTCCTAGGGACAGT |
| | loopF | CTGCATACGACGTGTCT |
| | loopB | ACCATCTATGACTGTACGCC |
| | rk1249.1 | |
| | F3 | TGACTTGGTACTGACCGACC |
| | B3 | CCCGGTGTACTTGCGATC |
| | FIP | TGCCTGCTGAGTGGTGACGATCCCTATGGCATTGCAGACG |
| | BIP | ACCAGTGGCTCAAGCCCTTCTCATGGAGCCGTTGTCCT |
| | loopF | GCCCTTCTTGGTGAGTTTGGC |
| | rk2208.1 | |
| | F3 | GAGAGACATGTCCGATTCCG |
| | B3 | GCCGATGTCATCAAGCTCAA |
| | FIP | TGTGACTTCCACGTCAAGCGTTGCAATCACCGACTTCCTCA |
| | BIP | GCGAGAAGCCCGTGTGCTTGTCACGATTTTCGGCCAGTT |
| | loopF | AGAGCTTTTCGCCAATCGACT |
| | rk2403.1 | |
| | F3 | GTCGAATACGGATGCGTTAC |
| | B3 | ACGCGAATCTTTTGGTTGG |
| | FIP | TGCCGCGCGCTTCAACAACTGTGGTCCGTTGAGGCCATA |
| | BIP | TGCCGTTTCCCGCGGTCGGTTGCAGCGAGAGGAT |

| LAMP primer sets | | |
|---|---|---|
| | loopF | ACTGGGCGAGATAAA |
| rsfliC | | |
| | F3 | TTCAAGTTGCAGGTCACGT |
| | B3 | AGGTTGTTTTCAACCTGGCC |
| | FIP | GAGATGTTGGTATTGAGGCTGAGCAAGCATTCACTCGGGCA |
| | BIP | GCCTGACCACGACCCTGAACAGGTACGAGTTCGCACCGT |
| | loopB | CGCAAACGCAAGGTATCCAGA |
| spa1 | | |
| | F3 | AATGACTCTCAAGCTCCAA |
| | B3 | CTTTGTTGAAATTGTTGTCAGC |
| | FIP | GCTCTTCGTTTAAGTTAGGCATGTTTGCGCAACAAAATAAGTTCA |
| | BIP | AAGTCTTAAAGACGATCCAAGCCTTCGGTGCTTGAGATTCG |
| | loopB | AGCACTAACGTTTTAGGTGAAGC |
| Se01 | | |
| | F3 | GGCGATATTGGTGTTTATGGGG |
| | B3 | TGAACCTTTGGTAATAACGATAAACTG |
| | FIP | CTGGTACTGATCGATAATGCCAAGTTTTTCAACGTTTCCTGCGG |
| | BIP | GATGCCGGTGAAATTATCGCACAAAACCCACCGCCAGG |
| | loopF | GACGAAAGAGCGTGGTAATTAAC |
| | loopB | GGGCAATTCGTTATTGGCG |
| Assimilating Probes | | |
| | lambda phage F probe | |
| | | 5'- fluorophore - ACGCTGAGGACCCGGATGCGAATGCGGATGCGGATGCCGACTGCATACGAC GTGTCT -3' |

(continued)

| Assimilating Probes | | |
|---|---|---|
| lambda phage B probe | | |
| | | 5'- fluorophore - ACGCTGAGGACCCGGATGCGAATGCGGATGCGGATGCCGAACCATCTATGAC TGTACGCC -3' |
| rk1249.1 F probe | | |
| | | 5'- fluorophore - ACGCTGAGGACCCGGATGCGAATGCGGATGCGGATGCCGAGCCCTTCTTGGT GAGTTTGGC -3' |
| rk2208.1 F probe | | |
| | | 5'- fluorophore - ACGCTGAGGACCCGGATGCGAATGCGGATGCGGATGCCGAAGAGCTTTTCGC CAATCGACT -3' |
| rk2403.1 F probe | | |
| | | 5'- fluorophore - ACGCTGAGGACCCGGATGCGAATGCGGATGCGGATGCCGAACTGGGCGAGA TAAA -3' |
| rsfliC F probe | | |
| | | 5'- fluorophore - ACGCTGAGGACCCGGATGCGAATGCGGATGCGGATGCCGAGACATGACGGC TCCTATATTCCC -3' |
| rsfliC B probe | | |
| | | 5'- fluorophore - ACGCTGAGGACCCGGATGCGAATGCGGATGCGGATGCCGACGCAAACGCAA GGTATCCAGA -3' |
| Se01 F probe | | |

(continued)

| | | |
|---|---|---|
| **Assimilating Probes** | | |
| | | 5'- fluorophore - ACGCTGAGGACCCGGATGCGAATGCGGATGCGGATGCCGAGACGAAAGAGC GTGGTAATTAAC -3' |
| | Se01 B probe | |
| | | 5'- fluorophore - ACGCTGAGGACCCGGATGCGAATGCGGATGCGGATGCCGAGGGCAATTCGTT ATTGGCG -3' |
| | spa1 B probe | |
| | | 5'- fluorophore - ACGCTGAGGACCCGGATGCGAATGCGGATGCGGATGCCGAAGCACTAACGT TTTAGGTGAAGC -3' |
| | Quencher 01 probe | |
| | | 5'- TCGGCATCCGCATCCGCATTCGCATCCGGGTCCTCAGCGT - Black Hole Quencher -3' |
| | Quencher 02 probe | |
| | | 5'- ACGCAGGGATCTGGCACGGATGCTCAGACTCTCGCGCCGT - Black Hole Quencher -3' |
| **Molecular beacons** | | |
| | rsfliC F-loop beacon | |
| | | 5'- FAM - CCTGCGACATGACGGCTCCTATATTCCCGCAGG - Black Hole Quencher1 -3' |
| | rsfliC B-loop beacon | |
| | | 5'- FAM - CCTGCCGCAAACGCAAGGTATCCAGAGCAGG - Black Hole Quencher1 -3' |

EP 2 585 617 B1

14

Several parameters pertaining to the assimilating probes have been identified which, in some embodiments, may significantly improve the performance of the assimilating probes as compared to other detection techniques. These parameters include the ratio of the amount of the fluorescent probe to the quencher probe, the manner in which the assimilating probe is added to the LAMP reaction mixture, and the total quantity of the assimilating probe competing for annealing sites on the single-stranded loop of the LAMP amplicon. For example, while the presence of the assimilating probes in the LAMP reaction mixture may cause the LAMP reaction rate to decrease, when the parameter values are within selected ranges, the degree to which the presence of the assimilating probes in the LAMP reaction mixture inhibits the LAMP reaction rate can become negligible.

With respect to the fluorescent probe and the quencher probe, the ratio of the fluorescent probe to the quencher probe is selected to be less than about 1:1 (e.g., higher concentrations of the quencher probe than fluorescent probe). Examples of such ratios may include, but are not limited to, less than about 1:1.1, less than about 1:1.2, less than about 1:1.3, less than about 1:1.4, less than about 1:1.5, less than about 1:1.6, less than about 1:1.7, less than about 1:1.8, less than about 1:1.9, and smaller. Examples of such ratios may further include, but are not limited to, less than about 1:2, less than about 1:3, less than about 1:4, less than about 1:5 and smaller.

Beneficially, ratios within this range have been found to reduce the degree to which the presence of the assimilating probe inhibits the rate of the LAMP reaction and reduce the degree of background fluorescence confounding detection. For example, as discussed in greater detail below, Example 4 illustrates that ratios of the fluorescent probe to the quencher probe of approximately 1:1 (e.g., molecular zippers) provide substantially no increase in fluorescence signal after incubation at about 65°C for about 120 minutes, with a high fluorescence background, indicative of incomplete assembly of fluorescent strands in the molecular zippers at the reaction temperature.

In contrast, when employing fluorescent probe to quencher probe ratios of approximately 1:2, large quantities of amplicon were observed after an incubation of about 60 minutes at about 65°C (Example 6). In some embodiments, similar results may be obtained for ratios less than about 1:2.

In further embodiments, the manner of mixing the strands of the assimilating probe into the LAMP reaction mixture may be varied to increase the speed of the LAMP reaction and, thus reducing the time needed to generate an amount of amplified target DNA sufficient for detection. The fluorescent probe and the quencher probe may in an unhybridized state with respect to each other when added to the LAMP reaction mixture. That is to say, the fluorescent probe and the quencher probe may be added separately to the LAMP reaction mixture. This is in contrast to the case of the molecular zippers, where the fluorescent and quencher probes are hybridizing together and then added to the LAMP reaction mixture. In certain embodiments, the fluorescent probes and quencher probes of the present disclosure may be added to the LAMP reaction mixture concurrently with one another or at different times.

It is observed that adding the fluorescent probe and the quencher probe directly to the LAMP reaction mixture individually, as opposed to adding a double-stranded assimilating probe structure (comprising the fluorescent probe and the quencher probe) to the LAMP reaction mixture, the LAMP reaction rate is relatively uninhibited, resulting in faster indication of a positive reaction (Example 5).

In additional embodiments, it has been identified that the polymerase concentration may be varied to influence the rate of LAMP reaction and, thus decrease the time needed to identify a positive reaction. For example, in one embodiment, polymerase concentrations may be greater than or equal to about 8U, greater than or equal to about 16U, greater than or equal to about 24U, greater than or equal to about 32U, etc. of the polymerase may be used to increase the LAMP reaction speed and decrease detection time.

For example, as discussed in greater detail below (Example 8), fluorescence was detected after about 20 minutes using the rk2208.1 primer set and about 8U *Bst* DNA polymerase. In contrast, when doubling the amount of *Bst* DNA polymerase from about 8U to about 16U, fluorescence was detected after about 10 minutes. This result indicates that the LAMP reaction speed is roughly doubled with a doubling of the polymerase concentration. Further increasing the polymerase concentration is anticipated to decrease the detection time. It is also believed that the additional gains in speed are approximately linear up to about the point where the rate limiting primer annealing rates become limiting as they are exceeded by the enzyme catalyzed polymerization rate.

The total amount of the assimilating probe within the reaction mixture may also be varied to influence the speed of the LAMP reaction and the onset of observable fluorescence. It has been identified that the detection time is significantly reduced by increasing the amounts of the fluorescent and quencher probes that are added to the reaction mixture (see, e.g., Example 10). For example, as discussed in Example 8, the amount of fluorescence probe added to the LAMP reaction mixture may be greater than about 0.08 $\mu$M, greater than or equal to about 0.4 $\mu$M, greater than or equal to about 0.8 $\mu$M, etc. and respective concentrations of the quencher probes may be greater than or equal to about 0.16 $\mu$M, greater than or equal to about 1.6 $\mu$M, etc.

The ratio of fluorescent probe to the quencher probe is less than about 1:1. Examples of such ratios may include, but are not limited to, less than about 1:1.5, less than about 1:2, less than about 1:2.5, less than about 1:3, less than about 1:3.5, less than about 1:1.4, less than about 1:1.4.5, less than about 1:5, less than about 1:5.5, less than about 1:1.60, less than about 1:1.65, less than about 1:1.70, less than about 1:1.75, less than about 1:1.80, less than about 1:8.5, less than about 1:9, less than about 1:9.5, and smaller. Examples of such ratios may further include, but are not limited to, about 1:2, about 1:3, about 1:4, about 1:5 and smaller.

In certain embodiments, the amount of the fluorescent probe and the quencher probe may be kept as low as possible while still providing detectable levels of fluorescence when positive amplification of DNA by LAMP takes place in the LAMP reaction mixture. In this manner, detection may still be performed while substantially eliminating reduction in the LAMP reaction rate due to the presence of the assimilating probe. In certain embodiments, the amount of the fluorescent probe may be within the range between about 0.01 to about 0.4 $\mu$M. In further embodiments, the amount of the quencher probe may selected be within the range between about 0.02 to about 0.8 $\mu$M. In other embodiments, the total amount of the assimilating probe may be within the range between about 0.03 $\mu$M to about 1.2 $\mu$M.

As discussed in greater detail below in the examples, it has been determined in Example 10 that higher quantities of the assimilating probe in the LAMP reaction mixture resulted in longer delays in the onset of fluorescence detection. This may occur as assimilating probes inhibit the speed of the LAMP reaction when present in large amounts. For example, a 10 fold increase in fluorescent probe and quencher probe concentrations (e.g., about 0.08 $\mu$M and about 0.16 $\mu$M to about 0.8 $\mu$M and 1.6 $\mu$M, respectively) provides an approximately 6 fold increase in fluorescence detection time, from about 20 minutes to about 120 minutes.

## EXAMPLES

In the examples below, embodiments of the assimilating probes are tested to assess their ability to monitor LAMP reactions. Comparisons are also performed between embodiments of the assimilating probe and detection techniques employing molecular zippers, molecular beacons, and intercalating dyes.

## LAMP Primers

A variety of different LAMP primer sets were used to evaluate the performance of the assimilating probes: lambda phage LAMP, rk1249.1, rk2208.1, rk2403.1, rsfliC and, Se01, and Spa1. The rsfliC LAMP primer set is designed to target the *Ralstonia solanacearum* (Rs) flagellin subunit C (*fliC*) gene, which is present in most members of the species.

The rk1249.1 rk2208.1, and rk2403.1 primer sets are designed to target a DNA sequence region unique to a subgroup of Rs strains classified as Race3Biovar2 (R3B2). The Lambda phage DNA LAMP primer set is designed to target lambda phage DNA. The Se01 primer set is designed to target *Salmonella enterica.* The Spa1 LAMP primer set is designed to target *Staphylococcus aureus.*

In an embodiment, the LAMP primers may be obtained from a supplier. In other embodiments, the LAMP primers may be designed as derivitized. An example of such derivitzed LAMP primers may include, but are not limited to, those disclosed in Kubota, R., B. G. Vine, A. M. Alvarez, and D. M. Jenkins, "Detection of Ralstonia solanacearum by loop-mediated isothermal amplification," Phytopathology. 98(9):1045-1051 (2008). The sequence for each of the primer sets and assimilating probe strands may be found in Table 2.

In the experiments discussed below, the protocols of Kubota et al. were employed to selectively amplify different target sequences. In an embodiment, the DNA targets comprised one of bacteriophage lambda, race 3 biovar 2 strains of *Ralstonia solanacearum, Ralstonia solanacearum, Salmonella enterica,* and *Staphylococcus aureus* using sequence comparison information provided by co-authors Schell and Allen (data not shown).

In one embodiment, LAMP reactions were performed in approximately 25 $\mu$l (total volume) reaction mixtures containing about 1:6 $\mu$M FIP and BIP, about 0.2 $\mu$M concentrations of the F3 and B3 primer, about 0.4 $\mu$M concentrations of the loop B primer, about 400 $\mu$M deoxynucleoside triphosphates (dNTPs), about 1.0 M betaine (e.g., Sigma-Aldrich Corp, St Louis, MO), 20 mM Tris-HCl (approximately pH 8.8), about 10 mM KCl, about 10 mM $(NH_4)_2SO_4$, about 6 mM $MgSO_4$, about 0.1% Triton X-100 and template DNA. The reactions were carried out in approximately 0.2 ml microtubes, using a thermal cycler for temperature control. The mixtures were heated to a temperature of about 95°C for about 5 min, then chilled on ice prior to addition of about 8 U *Bst* DNA polymerase large fragment (e.g., New England Biolabs, Inc., Beverly, MA).

In another embodiment, multiplexed real-time LAMP reactions were performed in 25 $\mu$l containing both Se01 LAMP and lambda phage LAMP primer sets at the following concentrations: 1.6 $\mu$M FIP and BIP; 0.2 $\mu$M F3 and B3, and; 0.4 $\mu$M loop F and B. Assimilating probes were contained in the reaction mixture at concentrations of 0.08 $\mu$M each of the FAM Se01 F probe, FAM Se01 B probe, and the lambda phage F probe, and 0.4 $\mu$M of the Quencher probe 01.

Other reagent concentrations were as follows: 400 $\mu$M dNTPs; 1.0 M betaine (Sigma-Aldrich Corp, St Louis, MO); 20 mM Tris-HCl (pH 8.8); 10 mM KCl, 10 mM $(NH_4)_2SO_4$; 8 mM $MgSO_4$; 0.1% Triton X-100; 8 U *Bst* DNA polymerase large fragment (New England Biolabs), and; template DNA (50 ng *Salmonella enterica* DNA and/or 5ng for lambda DNA).

For the real-time monitoring of LAMP reactions, the reactions were incubated at 65°C for 60 min and the fluorescence signals (fluorescein; $\lambda$ex/em = 500/530, Cy3; $\lambda$ex/em = 550/570) were measured every minute using the iQ5 Real-Time PCR Detection System (Bio-Rad Laboratories, Inc., Hercules, CA). Reactions were then terminated by heating to 80°C for 10 minutes. Quadruplicate reactions were run for each sample and fluorescence values were averaged for each set of samples for each point of time of reaction. For comparison of fluorescence data between channels, all fluorescence values were normalized to values where 0 and 1,000 RFU correspond respectively to the minimum and maximum fluorescence values observed for the averaged samples with greatest range of the fluorescence values on the given channel during the reaction.

After addition of the polymerase, the mixture was incubated at about 65°C for about 60 min. The reaction was terminated by heating to about 80 °C to denature the polymerase. The amplified products were electrophoresed at about 85V for about 90 min through about 2 % agarose gel (1 × Tris-acetate - EDTA), followed by staining with ethidium bromide, using appropriate size markers (e.g., Hyper ladder II; Bioline USA, Inc., Randolph, MA).

LAMP reactions were monitored real time using molecular zippers and assimilating probes. A molecular zipper was designed to target the loop region of a LAMP amplicon resulting from species specific primers (e.g., primers that resulted in amplification of DNA from all strains of *Ralstonia solanacearum.*). In an embodiment, the molecular zipper was produced by mixing about 2 μM of positive strand (fluorophore) and negative strand (quencher) in a buffer containing about 100 mM Tris-HCl (about pH 8.0) and about 10 mM EDTA (pH of about 8.0), heating at about 95°C for about 5 min, and cooling slowly to room temperature. About 0.04 μM concentration of the molecular zipper was used for the real-time LAMP reaction.

These molecular zippers were then added to reaction mixtures with the species-selective LAMP primers, the target DNA (e.g., *Ralstonia solanacearum*), and to negative controls (e.g., DNA from a lambda phage, or reaction mixtures containing no DNA).

To examine the ability of an assimilating probe to detect LAMP reactions, an assimilating probe designed to target the loop region of a LAMP amplicon resulting from species specific primers was also produced. In certain embodiments, the positive strand (fluorophore) and negative strand (quencher) were kept separate from each other and added directly to the LAMP mixture. The ratio of concentrations of the fluorophore and quencher was less than about 1:1, respectively.

The ability of the molecular zippers and assimilating probes to detect LAMP reactions occurring in LAMP reaction mixtures was determined from the fluorescence emitted from the reaction mixtures. For example, fluorescence was measured using a IQ5 real time PCR system (e.g., Bio-Rad, Hercules CA).

**Example 1 - Hardware Performance**

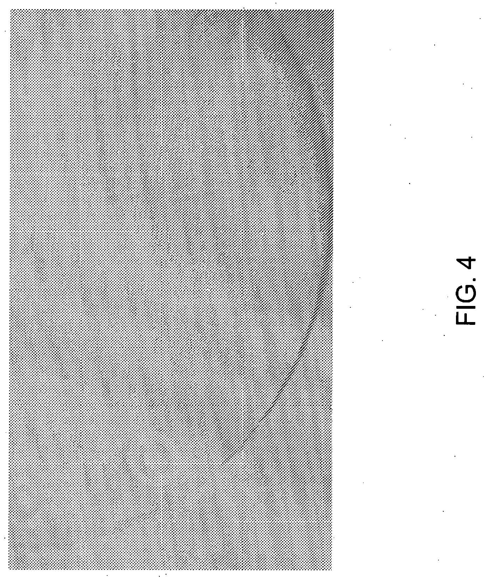

The custom fluorometer was used to successfully observe differences between LAMP products labeled with a molecular beacon targeting one of the loop regions, as compared to a negative control containing molecular beacons but no LAMP amplicon (data not shown). A custom printed CD in ABS with various size reaction wells is shown in Figure 4.

**Example 2 - Examination of Temperature Calibration and Control**

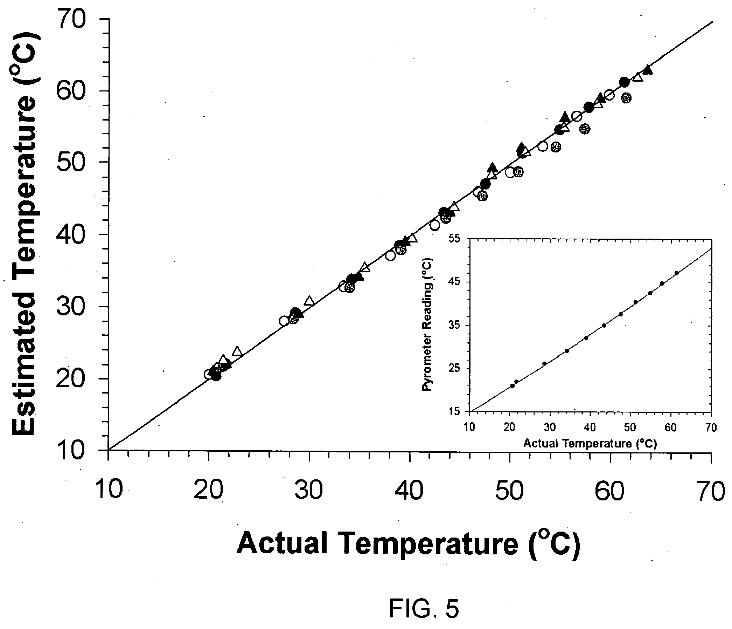

Temperature calibration was performed as discussed above to evaluate the calibration technique. Calibration measurements were conducted on reaction wells in patterned ABS and polycarbonate and yielded highly accurate, non-contact temperature measurements, despite the radiation losses through the well and transparent film. For example, Figure 5 illustrates corrected well temperatures estimated from a non-contact thermometer (e.g., pyrometer) compared to actual well temperatures measured by a type K thermocouple. Circles represent data measured from wells cut in polycarbonate, while triangles represent data measured from wells cut in ABS. Calibration data used to estimate coefficients for transmission of radiation through the well and reflection of ambient radiation from the well surface are illustrated in the inset of Figure 5. The standard error observed in the temperature an individually calibrated well on a polycarbonate CD was approximately 0.35°C. Applying the same calibration on a total of five wells, including some patterned into ABS, yielded a standard error in the measured temperature of about 0.93°C. The latter result suggests that, over the spectral range for thermal radiation at the observed temperatures, the emissivities of the ABS and polycarbonate are very similar.

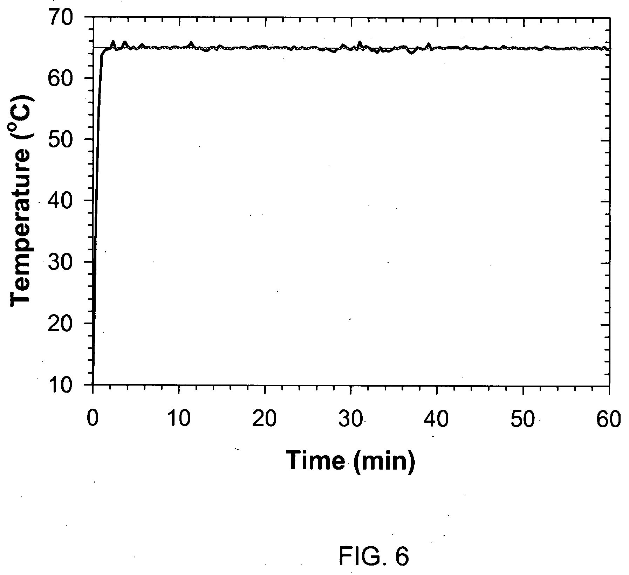

Figure 6 illustrates transient temperature measurements showing temperature control to a set point of about 65°C in a reaction well using only corrected non-contact temperature measurements. The data indicate that the set point was reached within about one minute. Furthermore, analysis of the data finds that the system root mean squared error after reaching the set point was about 0.27°C. These results indicate that the temperature within the reaction wells may be controlled to a high degree using embodiments of the disclosed calibration process.

In order to improve the range and speed of temperature control, DNA may be denatured prior to LAMP. In one embodiment, this may be performed using laser of higher power than that used to heat the well In further embodiments, the design of the reaction well may be altered to limit its thermal mass and improve the insulation.

**Example 3 -LAMP Reaction Setup**

*a) Bacterial Strains and DNA Purification*

Rs strains GMI1000 (R1B3) and UW551 (R3B2) were grown on modified tetrazolium chloride (TZC) agar medium according to Norman and Alvarez (Norman, D., and Alvarez, A. M., "A rapid method for presumptive identification of Xanthomonas campestris pv. dieffenbachiae and other xanthomonads," Plant Disease, 73, 654-658 (1989). The $R_s$ strains were incubated for about 48-72 h at about 28°C. DNAs were purified from these cells with the Wizard® Genomic DNA Purification Kit, (Promega Corp., Madison, WI) according to the manufacturer's instructions and quantified spectrophotometrically. For lambda DNA LAMP reaction, purified lambda DNA was purchased (New England Biolabs, Inc., Beverly, MA).

A *Staphylococcus aureus* strain was further grown using a Lysogeny broth (LB) medium and was incubated for about 18-24h at about 38°C. The cells were suspended in ddH20 and heat-killed at 100°C for 15min.

In embodiments of multiplexed detection, a *Salmonella enterica* strain was further grown on xylose-lysine-desoxycholate (XLD) agar medium (Zajic-Satler and Gragas, 1977) and incubated for 18-24h at 38°C. DNA was purified from these cells with the Wizard® Genomic DNA Purification Kit, (Promega Corp., Madison, WI) according to the manufacturer's instructions and quantified spectrophotometrically. For lambda phage LAMP reaction, purified lambda DNA was purchased (New England Biolabs, Inc., Beverly, MA).

*b) LAMP Reactions*

In embodiments of assimilating probes for single amplification detection, except where otherwise noted, LAMP reactions were performed using LAMP reaction mixtures having a total volume of 25 μl. In certain embodiments, the reaction mixtures comprised about 1.6 μM FIP and BIP, about 0.2 μM of the F3 and B3 primer, and about 0.4 μM concentrations of the loop primer.

Other regent concentrations in the LAMP reaction mixture were as follows: about 400 μM dNTPs, about 1.0 M betaine (Sigma-Aldrich Corp, St Louis, MO), about 20 mM Tris-HCl (pH about 8.8), about 10 mM KCl, about 10 mM $(NH_4)_2SO_4$, about 6 mM MgSO$_4$, about 0.1 % Triton X-100 and about 50 ng (or about 50pg for lambda DNA) of template DNA.

For rsfliC LAMP primer set, purified genomic DNA of GMI1000 strain was used as the positive control, and for rk2208.1 LAMP primer set DNA of UW551 strain was used as the positive control. The mixtures were heated to about 95°C for about 5 min, then chilled on ice prior to addition of about 8 U *Bst* DNA polymerase large fragment (New England Biolabs). Immediately after addition of the polymerase, the reaction was incubated at about 65°C for about 60 min and then the reaction was terminated by heating to about 80°C.

For embodiments of assimilating probes for multiplexed amplification detection, except where otherwise noted, LAMP reactions were performed using LAMP reaction mixtures having a total volume of 25 μl. In certain embodiments, the reaction mixtures comprised about 1.6 μM FIP and BIP, about 0.2 μM F3 and B3, and about 0.4 μM loop F and B. Assimilating probes were also contained in the reaction mixture at concentrations of about 0.08 μM for each of the FAM Se01 F probe, the FAM Se01 B probe, and the lambda phage F probe, and about 0.4 μM of the Quencher probe 01.

Other reagent concentrations in the LAMP reaction mixture were as follows: 400 μM dNTPs; 1.0 M betaine (Sigma-Aldrich Corp, St Louis, MO); 20 mM Tris-HCl (pH 8.8); 10 mM KCl, 10 mM $(NH_4)_2SO_4$; 8 mM MgSO$_4$; 0.1% Triton X-100; 8 U *Bst* DNA polymerase large fragment (New England Biolabs), and; template DNA (50 ng *Salmonella enterica* DNA and/or 5ng for lambda DNA).

For the real-time monitoring of LAMP reactions, the reactions were incubated at 65°C for 60 min and the fluorescence signals (fluorescein; λex/em = 500/530, Cy3; λex/em = 550/570) were measured every minute using the iQ5 Real-Time PCR Detection System (Bio-Rad Laboratories, Inc., Hercules, CA). Reactions were then terminated by heating to 80°C for 10 minutes. Quadruplicate reactions were run for each sample and fluorescence values were averaged for each set of samples for each point of time of reaction.

For comparison of fluorometric data between channels, all fluorescence values were normalized to values where 0 and 1,000 RFU correspond respectively to the minimum and maximum fluorescence values observed for the averaged samples with greatest range of the fluorescence values on the given channel during the reaction.

The amount of template DNA added to the reaction mixture was varied, depending on the LAMP primers used. For example, in the case of Lambda phage primer, about 5 ng *Bacteriophage lambda* DNA was added. In the case of rk1249.1, rk2208, and rk2403.1 primers, about 50 g *Ralstonia solanacearum* Race3 Biovar2 DNA was added. In the case of rsfliC primer, about 50 g *Ralstonia solanacearum* DNA was added. In the case of Se01 primer, about 50 ng *Salmonella enterica* DNA was added.. In the case of Spa1 primer, about 1.0 μl of heat-killed cells of *Staphylococcus aureus* DNA was added.

**c)** *Design of Assimilating Probes for LAMP Reactions.*

In embodiments of assimilating probes for single amplification detection, the fluorescent strand of the assimilating probe was designed to contain rsfliC loopB or rk2208.1 loopF primer sequences at the 3' end, and a sequence complementary to the quencher probe at the 5' end. A fluorescent molecule (e.g. fluorescein) was conjugated to the 5' end of the fluorescent probes, such that when annealed to the quenching strand in the assimilating probe a quencher molecule (e.g. BlackHoleQuecher-1) conjugated to the 3' end of the quencher strand effectively quenches the fluorescence. The molecular structure/ sequence of each primer and probe is summarized in Table 1.

In embodiments of assimilating probes for multiplexed amplification detection, the fluorescent strand of the assimilating probe was designed to contain Se01 loopF, Se01 loopB or lambda phage loopF primer sequences at the 3' end, and a sequence complementary to the quencher probe at the 5' end. Fluorescein molecule was conjugated to the 5' end of the Se01 F probe and Se01 B probe, and Cy3 molecule was conjugated to the 5' end of the lambda phage F probe, such that when annealed to the quenching strand in the assimilating probe a quencher molecule (e.g. Black-HoleQuecher-1) conjugated to the 3' end of the quencher strand effectively quenches the fluorescence. The molecular structure/ sequence of each primer and probe is summarized in Table 2.

**Example 4 -Evaluation of Molecular Zippers for rsfliC LAMP Reactions**

To examine the performance of molecular zippers for monitoring the LAMP reactions in real-time, molecular zippers were formed following the protocol previously described by Yi, et al (Yi, J. Z., W. D. Zhang, and D. Y. Zhang, "Molecular Zipper: a fluorescent probe for real-time isothermal DNA amplification," Nucleic Acids Research, 34 (2006). The rsfliC LAMP primer set was used in this experiment and the fluorescent probe was designed to contain the rsfliC LAMP loopB primer sequence in 3' end side, as illustrated in Table 1. A final concentration of about 0.4 $\mu$M of molecular zipper was mixed with rsfliC LAMP reaction mixture and the mixture was incubated at about 65°C for about 120 min.

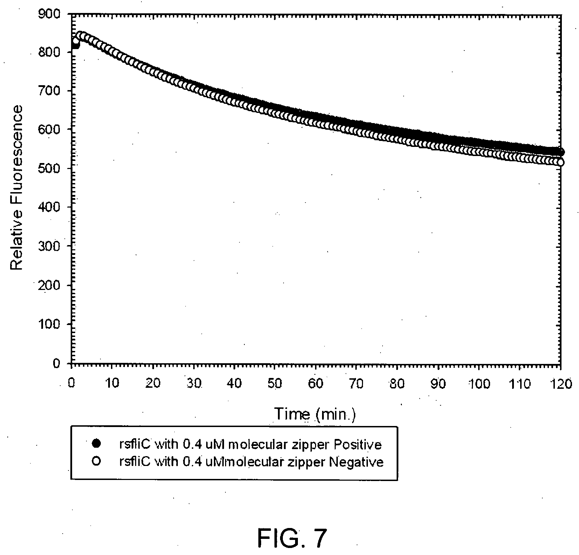

The fluorescence signal was observed using the iQ5 Real-Time PCR Detection System. Genomic DNA (50ng) of $R_s$ strain, GMI1000, was used for positive samples and double distilled water (ddH$_2$O) was used as a negative control. Both treatments were prepared in triplicate, and data from the replicates were averaged to generate an average fluorescence profile throughout the course of a given reaction (e.g. Figure 7).

As illustrated in Figure 7, no significant difference was observed in the fluorescence profiles of the two treatments (GMI1000 genomic DNA vs. ddH2O) using molecular zippers. Furthermore, no increase in the fluorescence signal was observed during the course of either reaction. Both treatments exhibited a substantially high background fluorescence signal, indicative of substantially incomplete assembly of the fluorescent strands into molecular zippers at the reaction temperature.

**Example 5 - Real-Time Monitoring of rsfliC LAMP Reaction Using Assimilating Probes**

To monitor rsfliC LAMP reactions in real-time without substantially sacrificing reaction speed, about 0.08 $\mu$M fluorescent probe and about 0.16 $\mu$M of the quencher probe were included directly in the reaction mix prior to the formation of the double stranded molecular zipper structure. The reaction mixtures were incubated at about 65°C for about 120 min and the fluorescence signal was observed using the iQ5 Real-Time PCR Detection System.

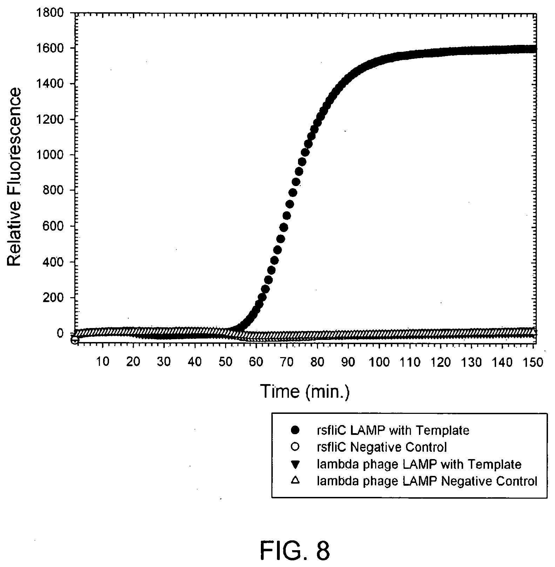

Figure 8 illustrates that the fluorescence signal of rsfliC LAMP reaction in the presence of assimilating probe precursors appears after about 30 min of incubation, substantially consistent with the rsfliC reaction speed as elucidated using molecular beacons (Experiment 6). Hybridizing the strands of the assimilating probe and adding the hybridized assimilating probe to the LAMP reaction mixture significantly interferes with rsfliC LAMP reaction, resulting in delayed indication of a positive reaction. However, by adding the fluorescent and quencher probes directly to rsfliC LAMP reaction mixture, the speed of rsfliC LAMP with probes was drastically improved, reflecting the speed of the uninhibited rsfliC LAMP reaction.

As double stranded DNA probe is in the condition of dynamic equilibrium at about 65°C, fluorescent and quencher probe molecules are constantly dissociating and reassociating, facilitating the interaction of probes with LAMP amplicons. To suppress background fluorescence under these conditions, the concentration of quencher probe should be higher than that of the fluorescent probe so that unquenched fluorescence only occurs when the latter is assimilated into the LAMP amplicon.

**Example 6 - Examination of Ratio of Fluorescence and Quencher Probes on LAMP Reaction Speed**

The ratio of the fluorescent and quencher probes of the molecular zippers were adjusted to provide assimilating probes for monitoring the rsfliC LAMP reaction. In order to reduce the background signal of fluorescent probe, the ratio of fluorescent probe to quencher probe was lowered to about 1:2. For monitoring the LAMP reaction, about 0.08 $\mu$M of

the resulting assimilating probe comprising about 0.08 of fluorescent probe and about 0.16 μM of quencher probe was mixed with rsfliC LAMP reaction mixture. In order to verify the sequence specificity of fluorescent and quencher probes, lambda DNA LAMP primer set was used with rsfliC assimilating probe as a control for the real-time LAMP.

Fluorescence signals associated with assimilation of the assimilation probe into the LAMP amplicon were observed only from the rsfliC LAMP reaction mixture with GMI1000 genomic DNA and not from the lambda DNA LAMP reaction. This observation demonstrates the sequence specificity of rsfliC assimilating probe.

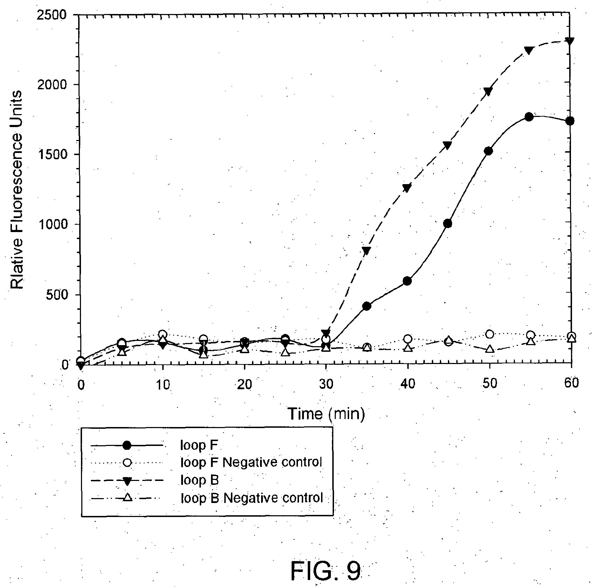

Agarose gel electrophoresis analysis was further performed to confirm the presence of both rsfliC and lambda DNA LAMP amplicons in the respective reactions. The fluorescence profile (Figure 9) indicated that the rsfliC LAMP reaction could be detected using the given concentration of assimilating probe but only after an incubation of about 60 min at about 65°C. In contrast, the rsfliC LAMP reaction in the absence of the assimilating probe typically resulted in large quantities of amplicon within about 60 minutes at about 65°C. This may be observed by the accumulation of precipitated magnesium pyrophosphate by-product of the polymerization reaction and probing the stopped reaction at various intervals with molecular beacons (see Experiment 7 below) or gel electrophoresis. This result suggests that the presence of assimilating probe may inhibit annealing of loop primers in the LAMP reaction, delaying its overall progress. Therefore, the total concentration of assimilating probe may be varied within a selected range to allow the LAMP reaction to proceed at full speed while still enabling real-time sequence specific detection.

**Example 7 - Measurement of speed of rsfliC LAMP Primer Set by Molecular Beacon.**

In order to determine the true speed of rsfliC LAMP reaction, molecular beacons as shown in Table 1 were designed to target rsfliC loopF and loopB regions. About 0.4 μM of the molecular beacon was added to rsfliC LAMP reactions and incubated at about 65°C for approximately 0, 5, 10, 15, 20, 25, 30, 35, 40, 45, 50, 60 minutes respectively. Each reaction was terminated by incubating at about 80°C for about 10 min. The intensity of fluorescence of rsfliC LAMP mixtures with molecular beacons was measured at about 25°C after terminating the reactions. To verify that molecular beacons did not retard the reactions, the stopped reactions at various intervals without molecular beacons were subjected to agarose gel electrophoresis in order to confirm the presence of rsfliC amplicons.

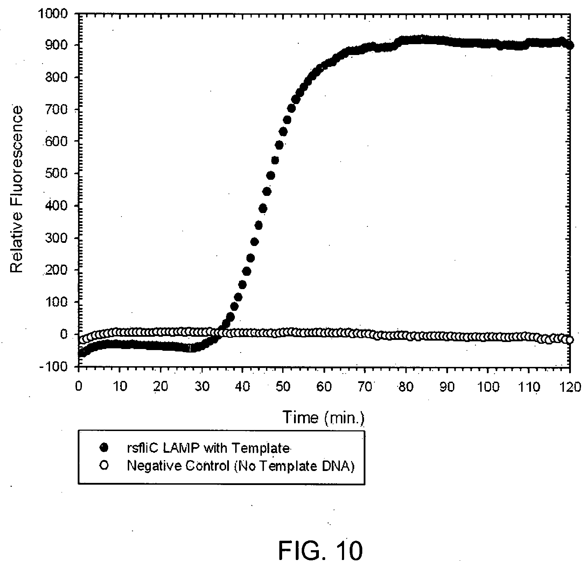

Figure 10 illustrates results for relative fluorescence as a function of time. The results of Figure 10 show that both molecular beacons for rsfliC loopF and B regions were successfully able to hybridize to their target regions of the rsfliC LAMP amplicon. The sequence specificity of both molecular beacons was also confirmed using lambda DNA LAMP reaction as a negative control (data not shown). Molecular beacon data further indicates that proliferation of detectable quantities of amplicon from the rsfliC LAMP reaction occurs within about 30 min of incubation and saturation occurs around about 55 min of incubation.

Agarose gel electrophoresis analysis of rsfliC reaction without molecular beacons also showed the appearance of faint bands starting at about 30 min (data not shown), correlating with the onset of fluorescence in the molecular beacon containing reactions. These results, compared to those from Experiment 6, support the conclusion that molecular zippers decrease the speed of rsfliC LAMP reaction significantly.

**Example 8 - Influence of Increased Polymerase Concentration on LAMP Reaction Speed**

rsfliC and R3B2 specific rk2208.1 LAMP primer sets were used to initiate LAMP reactions and were monitored in real time using the corresponding assimilating probes with the concentration of about 0.08 μM fluorescent probe and about 0.16 μM quencher probes. Assimilating probes were included directly in the reaction mix prior to the formation of the double stranded molecular zipper structure. To investigate the kinetic constraints of the reaction rate, reactions with each primer set were run using different amounts (about 8U or about 16U) of *Bst* DNA polymerase.

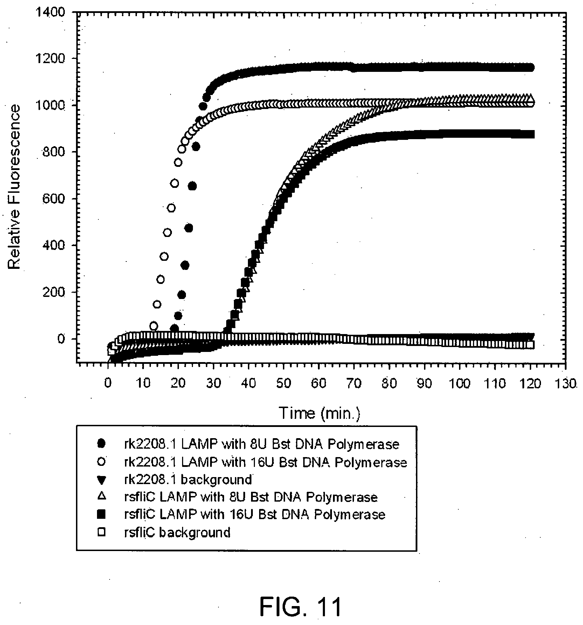

As illustrated in Figure 11, the LAMP reaction using the rk2208.1 primer set resulted in observable fluorescence increases within about 20 minutes when using about 8U of *Bst* DNA polymerase, and increases within about 10 minutes when using about 16U. The LAMP reaction with rsfliC LAMP primer set resulted in observable fluorescence increases within about 30 min regardless of *Bst* DNA polymerase concentration.

This result indicated that of the forward binding rate constant of the rate limiting primer annealing steps of the rk2208.1 reaction were significantly higher than the corresponding rates in the rsfliC reaction. This improved annealing rate was significant enough that proportional kinetic gains could be observed in the rk2208.1 reaction by increasing the polymerase activity. Additional gains may be observed by corresponding increases in the polymerase concentration in the reaction.

**Example 9 - Further examination of the Influence of *Bst* DNA polymerase Concentration with rk2208.1 LAMP on Detection Speed and Reaction Kinetics**

R3B2 specific rk2208.1 LAMP primer sets were used to initiate LAMP reactions and were monitored in real time using the corresponding assimilating probes with concentrations of about 0.08 μM fluorescent probe and about 0.16 μM quencher probe. Assimilating probes were included directly in the reaction mix prior to the formation of the double stranded molecular zipper structure. To investigate the kinetic constraints of the reaction rate, reactions with each primer set were run using different amounts, about 0 U, about 4 U, about 8 U, about 12 U, about 16 U, about 20 U, about 24 U, about 28 U, and about 32 U) of *Bst.* DNA.

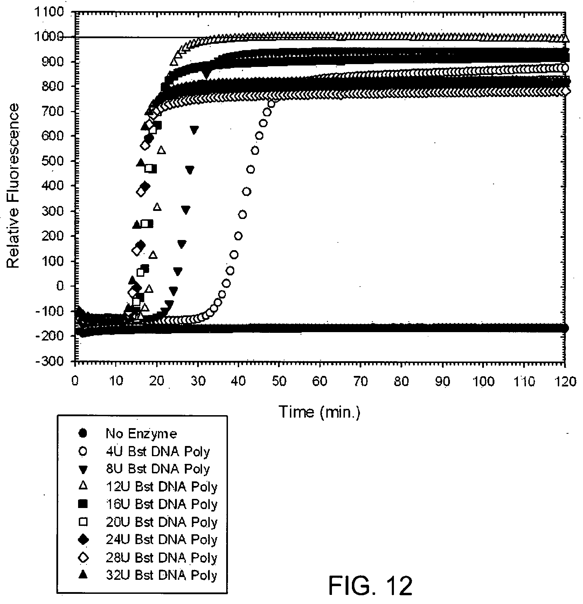

The LAMP reaction using the rk2208.1 primer set resulted in observable fluorescence increases within about 30 minutes when about 4 U of *Bst* DNA polymerase (Figure 12). Fluorescence was observed in a shorter time, after about 20 minutes, with an increase in polymerase concentration to about 8 U. Increasing the polymerase concentration to greater than about 15 U (e.g., about 15U, about 20 U, about 24 U, about 28 U, and about 32 U) resulted in observable fluorescence within about 10 to 15 minutes.

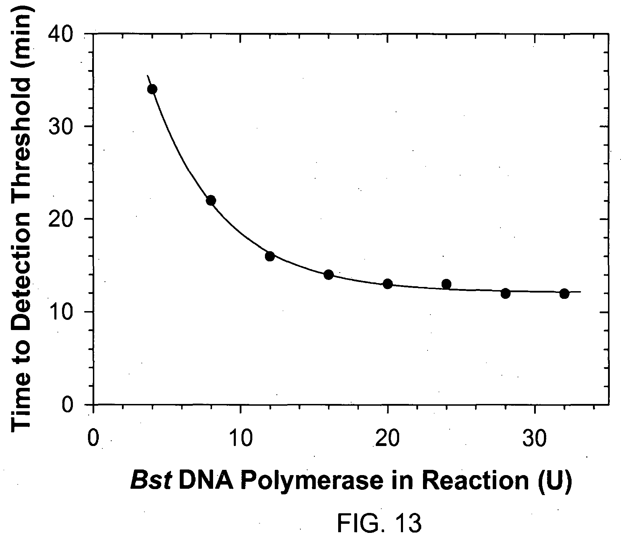

Increasing *Bst* DNA polymerase concentration further provides an increase in the time to reach maximum velocity. Figure 13 illustrates relationship with time to reach maximum velocity of fluorescence gain per minute.. The time to maximum velocity is decreased from about 40 minutes to about 15 minutes with increasing *Bst* DNA polymerase concentration from about 4 U to about 32 U.

**Example 10 - Examination of Assimilating Probe Composition for rk2208.1 LAMP Primer Set Without Loop Primer**

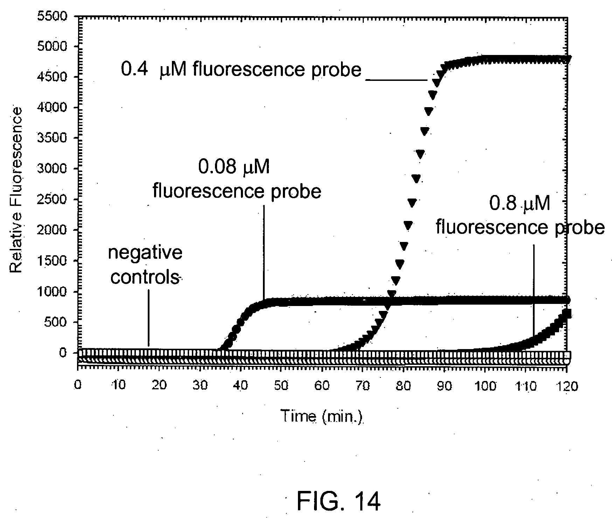

The rk2208.1 LAMP primer set, without loop primer, was used to determine the impact of the amounts of fluorescent probe and quencher probe on the time to detect fluorescence from the LAMP reaction mixture. Reactions were run using fluorescent probe concentrations of about 0.08 μM, about 0.4 μM, and about 0.8 μM, mixed respectively with quencher probe concentrations of about 0.16 μM, about 0.8 μM, and about 1.6 μM in the LAMP reaction, without loop primers. The results of these tests are illustrated in Figure 14. The circles represent low probe concentrations (about 0.08 μM fluorescent probe and about 0.16 μM quencher probe), the triangles are medium probe concentrations (about 0.4 μM fluorescent probe and about 0.8 μM quencher probe), and rectangles are high medium probe concentrations (about 0.8 μM fluorescent probe and about 1.6 μM quencher probe). Shaded symbols are reactions including template DNA and open symbols are negative control (ddH$_2$O).

As illustrated in Figure 14, generally, the presence of larger quantities of the assimilating probe in a reaction mix resulted in longer delays in the onset of observable fluorescence increase. This result confirmed that higher concentrations of assimilating probes may interfere the speed of rk2208.1 LAMP reaction. This experiment also demonstrates that the assimilating probe can be incorporated into the LAMP amplicon even in the absence of the LOOP primer. These results further suggest that the reaction inhibition by the assimilating probe affects steps in the LAMP process, aside from annealing and extension of the loop primer.

**Example 11 - Examination of Assimilating Probe Composition for rk2208.1 LAMP Primer Set With Loop Primer**

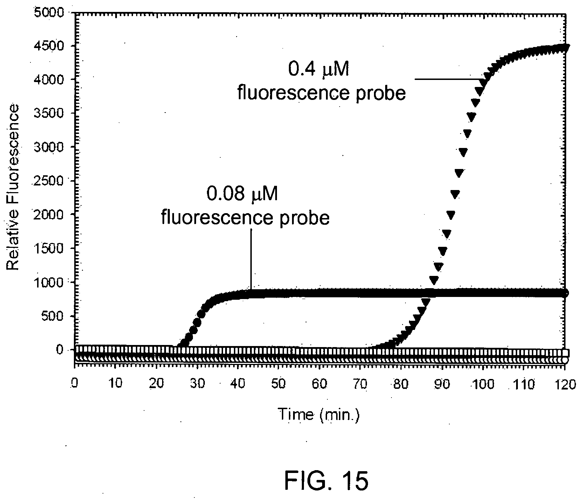

To determine the effect of assimilating probe composition on the LAMP reaction including loop primers, Experiment 10 was repeated after including loop primers in the reactions. The results of the experiment are illustrated in Figure 15, where circles represent low probe concentrations (about 0.08 μM fluorescent probe and about 0.16 μM quencher probe), triangles are medium probe concentrations (about 0.4 μM fluorescent probe and about 0.8 μM quencher probe), and rectangles are high medium probe concentrations (about t0.8 μM fluorescent probe and about 1.6 μM quencher probe). Shaded symbols are reactions including template DNA and open symbols are negative control (ddH$_2$O).

As illustrated in Figure 15, assimilation probes for rk2208.1 at the lowest concentrations tested (about 0.08 μM fluorescent probe and about 0.16 μM quencher probe) resulted in fluorescence signals after about 20 min. At a medium concentration (about 0.4 μM fluorescent probe and about 0.8 μM quencher probe) resulted in increased fluorescence after about 70 minutes. Reactions including the highest assimilation probe concentrations (about 0.8 μM fluorescent probe and about 1.6 μM quencher strand) did not result in increased fluorescence at any point during the about 120 minute reaction. This result confirms that higher concentrations of assimilation probes interferes with the speed of rk2208.1 LAMP reaction, and that the presence of rk2208.1 loopF primer enhances the speed of the reaction.

**Example 12 - Evaluation of Influence of Intercalating dye (EvaGreen) on LAMP Reaction and Speed.**

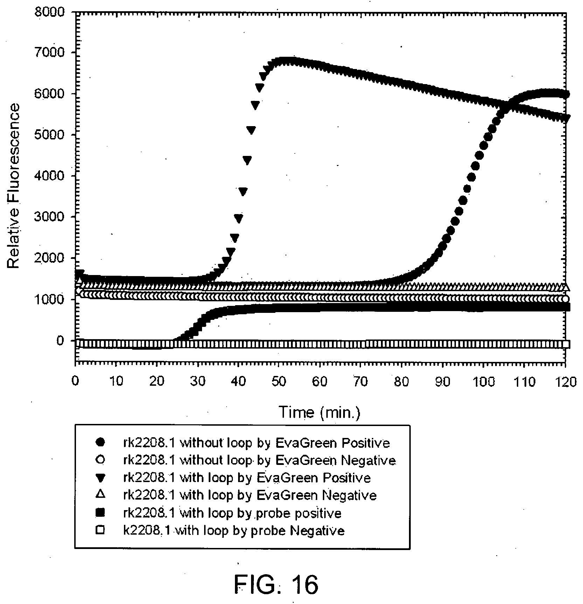

In order assess the influence of intercalating dye (EvaGreen) on the LAMP reaction, EvaGreen was added to rk2208.1 LAMP reaction mixture (with and without rk2208.1 loopF primer), and reactions were observed by measuring fluorescence signal. Time for onset of increased fluorescence under these conditions was compared to the speed of rk2208.1 LAMP reaction with optimal assimilating probe composition. The results of this experiment are illustrated in Figure 16.

rk2208.1 LAMP reaction (with and without rk2208.1 loopF primer) with EvaGreen showed fluorescence signal after incubation of about 30 and about 80 min respectively. Rk2208.1 LAMP reaction with assimilating probe showed reaction within about 20 min. These results suggest that the intercalating dye significantly inhibits the rk2208.1 LAMP reaction. EvaGreen might be interfering with the efficiency of *Bst* DNA polymerase, since this enzyme has strand-displacement activity and the intercalating dye stabilizes double-stranded DNA.

**Example 13 - Examination of Assimilating Probe Performance for Simultaneous LAMP Based Detection of *Salmonella enterica* and Bacteriophage lambda genomic DNAs**

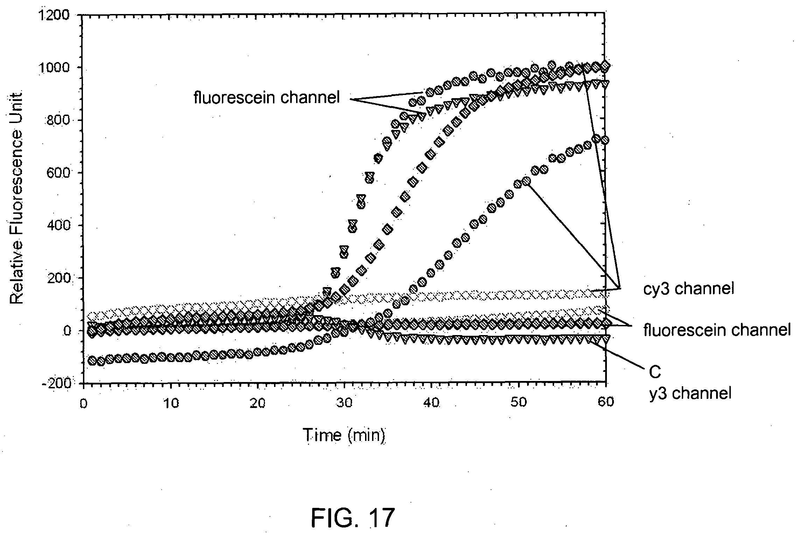

Figure 17 is a plot of relative fluorescence versus time for LAMP reactions amplifying *Salmonella enterica* and bacteriophage lambda genomic DNAs. Fluorescence values were measured on the fluorescein channel ($\lambda$ex/em = 500/530- probe label for Se01F) and on the cy3 channel ($\lambda$ex/em = 550/570- probe label for lambda phage F). Samples containing both *Salmonella enterica* and bacteriophage lambda genomic DNAs are represented by solid circles (●/●), negative controls with no template DNA are represented by open circle (○/○), samples with only *Salmonella enterica* genomic DNA are represented by solid triangles (▼/▼), and those with only bacteriophage lambda genomic DNA are represented by solid diamonds (♦/♦).

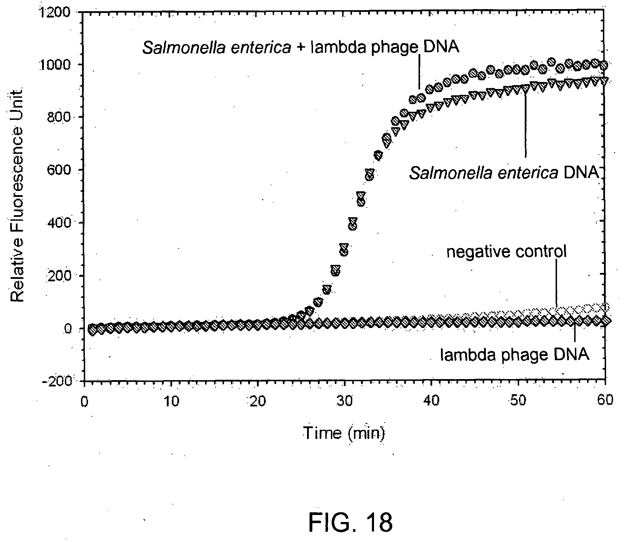

Figure 18 is a plot of relative fluorescence versus time for LAMP reactions amplifying *Salmonella enterica* and/or bacteriophage lambda genomic DNAs. Fluorescence values were measured on the fluorescein channel ($\lambda$ex/em = 500/530- probe label for Se01F) during multiplexed detection using assimilating probes. Samples with both *Salmonella enterica* and bacteriophage lambda genomic DNAs are represented by solid circles (●), negative controls with ddH2O are represented by open circles (○), samples with only *Salmonella enterica* genomic DNA are represented by solid triangles (▼), and those with only bacteriophage lambda genomic DNA are represented by solid diamonds (♦).

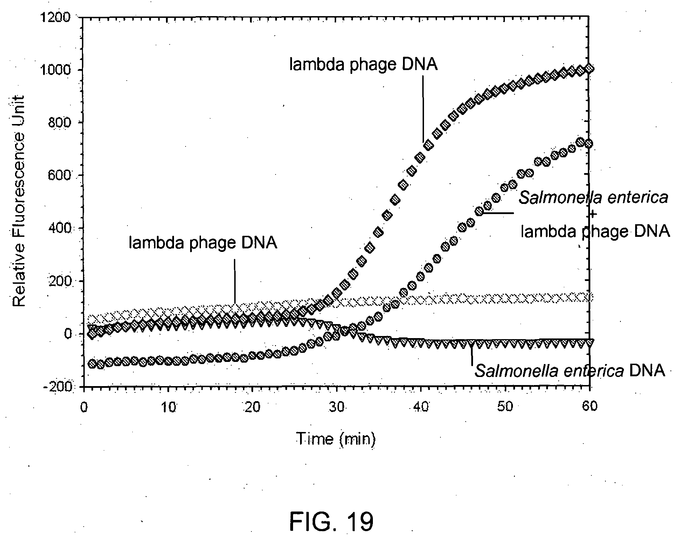

Figure 19 is a plot of relative fluorescence versus time for LAMP reactions amplifying *Salmonella enterica* and/or bacteriophage lambda genomic DNAs. Fluorescence values were measured on the cy3 channel ($\lambda$ex/em = 550/570- probe label for lambda phage F) during multiplexed detection with assimilating probes. Samples with both *Salmonella enterica* and bacteriophage lambda genomic DNAs are represented by solid circles (●), negative controls with ddH2O are represented by open circles (○), samples with only *Salmonella enterica* genomic DNA are represented by solid triangles (▼), and samples with only bacteriophage lambda genomic DNA are represented by solid diamonds (♦).

Notably, in each of the examples of Figures 17, 18, and 19, by using unique sequence specific assimilating probes for individual pathogen with spectrally unique fluorophores, different pathogens can specifically be detected at the same time

In summary, embodiments of several powerful new technologies are disclosed which alone or in any combination, may allow much more rapid typing of closely related sub-populations of bacterial pathogens which, despite displaying very similar antigenic determinants, may vary considerably in host specificity, virulence, or other biological characteristics important for disease spread. The gene-based technologies may be readily transferred given sequence data to discriminate the various populations of interest. By using an isothermal approach to DNA replication, sensitivity and selectivity of detection is improved even in relatively large sample volumes without the added expense, complexity, and energy requirements of thermal cycling equipment.

The ability to control the process temperature for the LAMP reaction on a compact disc media using a laser of similar specifications to standard digital media and non-contact temperature measurements after correcting for transmission losses through the reaction well and reflection of ambient thermal radiation is demonstrated.

The ability to observe the progression of LAMP replication in real time using optical probes is also demonstrated. Parameters which influence the conditions for hybridization of the molecular probes and may result in improved speed and sensitivity of detection have been identified. These parameters may include the ratio of quencher to fluorescent probe, the manner of mixing the strands to create the assimilating probe, the polymerase concentration, and the total amount of assimilating probe within the LAMP reaction mixture.

The performance of a custom fluorometer with the CD based system suggests that the reaction can be monitored in real time on the CD media, and experiments to demonstrate this definitively are on-going. Integration of this non-contact control and detection system with centrifugal based sequencing of sample capture, wash, lysis, and DNA extraction may result in a rapid, user friendly system to allow detection of pathogens in the field and in animal and plant quarantine situations.

The scope of the present teachings should not be limited to the foregoing discussion, but should be defined by the appended claims.

## Claims

1. A method of monitoring LOOP-mediated isothermal amplification (LAMP) of a target DNA, comprising:

    contacting an assimilating probe and a DNA polymerase with a LAMP reaction mixture,

wherein the LAMP reaction mixture comprises the target DNA and one or more LAMP primers that hybridize with the target DNA,

wherein the assimilating probe comprises a first and second oligonucleotide strand, wherein the first oligonucleotide strand comprises a quencher probe at a 3' end and wherein the second oligonucleotide strand of the assimilating probe comprises a fluorophore at a 5' end;

wherein the ratio of the amount of the second oligonucleotide strand to the amount of the first oligonucleotide strand is less than 1:1; and

measuring fluorescence emitted by the LAMP reaction mixture that has been contacted with the assimilating probe and the DNA polymerase.

2. The method of claim 1, wherein the concentration of the DNA polymerase is greater than or equal to 8U.

3. The method of claim 1, wherein the assimilating probe is contacted with the LAMP reaction mixture before the DNA polymerase is contacted with the LAMP reaction mixture.

4. The method of claim 1, wherein the DNA polymerase is contacted with a LAMP reaction mixture comprising an assimilating probe.

5. The method of claim 1, wherein the target DNA comprises DNA from bacteriophage lambda, race 3 biovar 2 strains of Ralstonia solanacearum, Ralstonia solanacearum, Salmonella enterica, or Staphylococcus aureus.

6. Use of a composition comprising an assimilating probe for monitoring LOOP-mediated isothermal amplification (LAMP) of a target DNA, wherein the composition comprises:

a first oligonucleotide strand comprising a quencher probe at a 3' end of the first oligonucleotide strand; and
a second oligonucleotide strand comprising a fluorophore at a 5' end of the second oligonucleotide strand;
wherein the ratio of the amount of the second oligonucleotide strand to the first oligonucleotide strand is less than 1:1.

7. The method of claim 1 or the use of claim 6, wherein the fluorophore comprises fluorescein, cy3, cy5 or one or more quantum dots.

8. The method of claim 1 or the use of claim 6, wherein the quencher comprises DABCYL, TAMRA or a Black Hole Quencher.

9. The method of claim 1 or the use of claim 6, wherein the first oligonucleotide strand and the second oligonucleotide strand are not hybridized with each other.

10. The method of claim 1 or the use of claim 6, wherein the amount of the first oligonucleotide strand is within the range between 0.02 to 0.8 µM.

11. The method of claim 1 or the use of claim 6, wherein the amount of the second oligonucleotide strand is within the range between 0.01 µM to 0.4 µM.

12. The use of claim 6, wherein the first and second oligonucleotides are complementary except over an overhanging unmatched segment of the second oligonucleotide strand.

13. A method of detecting the presence or absence of a target DNA comprising:

contacting an assimilating probe and a DNA polymerase with a LAMP reaction mixture,
wherein the LAMP reaction mixture comprises a sample to be tested for the presence of a target DNA and one or more LAMP primers capable of amplifying the target DNA,
wherein the assimilating probe comprises a first and second oligonucleotide strand, wherein the first oligonucleotide strand comprises a quencher probe at a 3' end and wherein the second oligonucleotide strand of the assimilating probe comprises a fluorophore at a 5' end;
wherein the ratio of the amount of the second oligonucleotide strand to the amount of the first oligonucleotide strand is less than 1:1; and
detecting the presence or absence of the target DNA.

**14.** The method of claim 13, wherein detecting comprises measuring fluorescence emitted by the LAMP reaction mixture that has been contacted with the assimilating probe and the DNA polymerase.

**15.** The method of claim 13 comprises:

simultaneously detecting the presence or absence of different target DNAs,
wherein the LAMP reaction mixture comprises more than one LAMP primers, and the more than one LAMP primers capable of amplifying the target DNA are capable of amplifying different target DNAs,
wherein multiple assimilating probes are contacted with the LAMP reaction mixture.

**Patentansprüche**

**1.** Verfahren zur Überwachung der LOOP-vermittelten isothermischen Amplifikation (LAMP) einer Ziel-DNA, umfassend:

Kontaktieren einer assimilierenden Sonde und einer DNA-Polymerase mit einem LAMP-Reaktionsgemisch, wobei das LAMP-Reaktionsgemisch die Ziel-DNA und ein oder mehrere LAMP-Primer, die mit der Ziel-DNA hybridisieren, umfasst,
wobei die assimilierende Sonde einen ersten und einen zweiten Oligonukleotid-Strang umfasst,
wobei der erste Oligonukleotid-Strang eine Quencher-Sonde an einem 3'-Ende umfasst und wobei der zweite Oligonukleotid-Strang der assimilierenden Sonde ein Fluorophor an einem 5'-Ende umfasst;
wobei das Verhältnis der Menge des zweiten Oligonukleotid-Strangs zu der Menge des ersten Oligonukleotid-Strangs weniger als 1:1 beträgt; und
Messen der Fluoreszenz, die durch das LAMP-Reaktionsgemisch, das mit der assimilierenden Sonde und der DNA-Polymerase kontaktiert worden ist, ausgesandt wird.

**2.** Verfahren gemäß Anspruch 1, wobei die Konzentration der DNA-Polymerase größer oder gleich 8 U beträgt.

**3.** Verfahren gemäß Anspruch 1, wobei die assimilierende Sonde mit dem LAMP-Reaktionsgemisch kontaktiert wird, bevor die DNA-Polymerase mit dem LAMP-Reaktionsgemisch kontaktiert wird.

**4.** Verfahren gemäß Anspruch 1, wobei die DNA-Polymerase mit einem LAMP-Reaktionsgemisch, das eine assimilierende Sonde umfasst, kontaktiert wird.

**5.** Verfahren gemäß Anspruch 1, wobei die Ziel-DNA die DNA von Bakteriophage Lambda, Rasse 3/Biovar 2-Stämmen von Ralstonia solanacearum, Ralstonia solanacearum, Salmonella enterica oder Staphylococcus aureus umfasst.

**6.** Verwendung einer Zusammensetzung, umfassend eine assimilierende Sonde, zur Überwachung der LOOP-vermittelten isothermischen Amplifikation (LAMP) einer Ziel-DNA, wobei die Zusammensetzung umfasst:

einen ersten Oligonukleotid-Strang, umfassend eine Quencher-Sonde an einem 3'-Ende des ersten Oligonukleotid-Strangs; und
einen zweiten Oligonukleotid-Strang, umfassend ein Fluorophor an einem 5'-Ende des zweiten Oligonukleotid-Strangs;
wobei das Verhältnis der Menge des zweiten Oligonukleotid-Strangs zu dem ersten Oligonukleotid-Strang weniger als 1:1 beträgt.

**7.** Verfahren gemäß Anspruch 1 oder Verwendung gemäß Anspruch 6, wobei das Fluorophor Fluorescein, Cy3, Cy5 oder ein oder mehrere Quantenpunkte umfasst.

**8.** Verfahren gemäß Anspruch 1 oder Verwendung gemäß Anspruch 6, wobei der Quencher DABCYL, TAMRA oder einen Black Hole Quencher umfasst.

**9.** Verfahren gemäß Anspruch 1 oder Verwendung gemäß Anspruch 6, wobei der erste Oligonukleotid-Strang und der zweite Oligonukleotid-Strang nicht miteinander hybridisiert sind.

**10.** Verfahren gemäß Anspruch 1 oder Verwendung gemäß Anspruch 6, wobei die Menge des ersten Oligonukleotid-Strangs innerhalb des Bereichs von 0,02 bis 0,8 μM liegt.

11. Verfahren gemäß Anspruch 1 oder Verwendung gemäß Anspruch 6, wobei die Menge des zweiten Oligonukleotid-Strangs innerhalb des Bereichs von 0,01 bis 0,4 μM liegt.

12. Verwendung gemäß Anspruch 6, wobei die ersten und zweiten Oligonukleotide komplementär sind, mit Ausnahme eines überhängenden ungepaarten Segments des zweiten Oligonukleotid-Strangs.

13. Verfahren zum Nachweisen des Vorhandenseins oder der Abwesenheit einer Ziel-DNA, umfassend:

Kontaktieren einer assimilierenden Sonde und einer DNA-Polymerase mit einem LAMP-Reaktionsgemisch, wobei das LAMP-Reaktionsgemisch eine auf das Vorhandensein einer Ziel-DNA zu testende Probe und ein oder mehrere zum Amplifizieren der Ziel-DNA fähige LAMP-Primer umfasst, wobei die assimilierende Sonde einen ersten und einen zweiten Oligonukleotid-Strang umfasst, wobei der erste Oligonukleotid-Strang eine Quencher-Sonde an einem 3'-Ende umfasst und wobei der zweite Oligonukleotid-Strang der assimilierenden Sonde ein Fluorophor an einem 5'-Ende umfasst; wobei das Verhältnis der Menge des zweiten Oligonukleotid-Strangs zu der Menge des ersten Oligonukleotid-Strangs weniger als 1:1 beträgt; und Nachweisen des Vorhandenseins oder der Abwesenheit der Ziel-DNA.

14. Verfahren gemäß Anspruch 13, wobei das Nachweisen das Messen der Fluoreszenz umfasst, die durch das LAMP-Reaktionsgemisch, das mit der assimilierenden Sonde und der DNA-Polymerase kontaktiert worden ist, ausgesandt wird.

15. Verfahren gemäß Anspruch 13, umfassend:

gleichzeitiges Nachweisen des Vorhandenseins oder der Abwesenheit verschiedener Ziel-DNAs, wobei das LAMP-Reaktionsgemisch mehr als einen LAMP-Primer umfasst, und die mehr als einen zum Amplifizieren der Ziel-DNA fähigen LAMP-Primer zum Amplifizieren verschiedener Ziel-DNAs fähig sind, wobei mehrfache assimilierende Sonden mit dem LAMP-Reaktionsgemisch kontaktiert werden.

**Revendications**

1. Procédé de surveillance d'une amplification isotherme médiée par des boucles (LAMP) d'un ADN cible, comprenant :

- mettre en contact une sonde d'assimilation et une ADN polymérase avec un mélange de réaction LAMP, le mélange de réaction LAMP comprenant l'ADN cible et une ou plusieurs amorces LAMP qui s'hybrident avec l'ADN cible, la sonde d'assimilation comprenant un premier et un second brin oligonucléotidique, le premier brin oligonucléotidique comprenant une sonde extincteur à une extrémité 3' et le second brin oligonucléotidique de la sonde d'assimilation comprenant un fluorophore à une extrémité 5' ; le rapport de la quantité du second brin oligonucléotidique à la quantité du premier brin oligonucléotidique étant inférieur à 1:1 ; et - mesurer la fluorescence émise par le mélange de réaction LAMP qui a été mis en contact avec la sonde d'assimilation et l'ADN polymérase.

2. Procédé selon la revendication 1, dans lequel la concentration de l'ADN polymérase est supérieure ou égale à 8U.

3. Procédé selon la revendication 1, dans lequel la sonde d'assimilation est mise en contact avec le mélange de réaction LAMP avant que l'ADN polymérase ne soit mise en contact avec le mélange de réaction LAMP.

4. Procédé selon la revendication 1, dans lequel l'ADN polymérase est mise en contact avec un mélange de réaction LAMP comprenant une sonde d'assimilation.

5. Procédé selon la revendication 1, dans lequel l'ADN cible comprend de l'ADN provenant du bactériophage lambda, de souches race 3 biovar 2 de Ralstonia solanacearum, de Ralstonia solanacearum, de Salmonella enterica ou de Staphylococcus aureus.

**6.** Utilisation d'une composition comprenant une sonde d'assimilation pour la surveillance d'une amplification isotherme médiée par des boucles (LAMP) d'un ADN cible, la composition comprenant :

un premier brin oligonucléotidique comprenant une sonde extincteur à une extrémité 3' du premier brin oligonucléotidique ; et
un second brin oligonucléotidique comprenant un fluorophore à une extrémité 5' du second brin oligonucléotidique ;
le rapport de la quantité du second brin oligonucléotidique au premier brin oligonucléotidique étant inférieur à 1:1.

**7.** Procédé selon la revendication 1 ou utilisation selon la revendication 6, dans lequel ou laquelle le fluorophore comprend la fluorescéine, cy3, cy5 ou un ou plusieurs points quantiques.

**8.** Procédé selon la revendication 1 ou utilisation selon la revendication 6, dans lequel ou laquelle l'extincteur comprend DABCYL, TAMRA ou un Black Hole Quencher.

**9.** Procédé selon la revendication 1 ou utilisation selon la revendication 6, dans lequel ou laquelle le premier brin oligonucléotidique et le second brin oligonucléotidique ne sont pas hybridés l'un avec l'autre.

**10.** Procédé selon la revendication 1 ou utilisation selon la revendication 6, dans lequel ou laquelle la quantité du premier brin oligonucléotidique se situe dans la plage entre 0,02 et 0,8 $\mu$M.

**11.** Procédé selon la revendication 1 ou utilisation selon la revendication 6, dans lequel ou laquelle la quantité du second brin oligonucléotidique se situe dans la plage entre 0,01 $\mu$M et 0,4 $\mu$M.

**12.** Utilisation selon la revendication 6, dans laquelle les premier et second oligonucléotides sont complémentaires sauf sur un segment non apparié de dépassement du second brin oligonucléotidique.

**13.** Procédé de détection de la présence ou de l'absence d'un ADN cible, comprenant :

- mettre en contact une sonde d'assimilation et une ADN polymérase avec un mélange de réaction LAMP, le mélange de réaction LAMP comprenant un échantillon à tester pour la présence d'un ADN cible et une ou plusieurs amorces LAMP capables d'amplifier l'ADN cible,
la sonde d'assimilation comprenant un premier et un second brin oligonucléotidique, le premier brin oligonucléotidique comprenant une sonde extincteur à une extrémité 3' et le second brin oligonucléotidique de la sonde d'assimilation comprenant un fluorophore à une extrémité 5' ;
le rapport de la quantité du second brin oligonucléotidique à la quantité du premier brin oligonucléotidique étant inférieur à 1:1 ; et
- détecter la présence ou l'absence de l'ADN cible.

**14.** Procédé selon la revendication 13, dans lequel la détection comprend la mesure de la fluorescence émise par le mélange de réaction LAMP qui a été mis en contact avec la sonde d'assimilation et l'ADN polymérase.

**15.** Procédé selon la revendication 13, comprenant :

- détecter simultanément la présence ou l'absence de différents ADN cibles,

le mélange de réaction LAMP comprenant plus d'une amorce LAMP, et les plus d'une amorce LAMP capables d'amplifier l'ADN cible étant capables d'amplifier différents ADN cibles,
de multiples sondes d'assimilation étant mises en contact avec le mélange de réaction LAMP.

FIG. 1

FIG. 2

FIG. 3B

FIG. 3A

FIG. 4

FIG. 5

EP 2 585 617 B1

FIG. 6

EP 2 585 617 B1

FIG. 7

FIG. 8

FIG. 9

FIG. 10

Relative Fluorescence (y-axis: -100, 0, 100, 200, 300, 400, 500, 600, 700, 800, 900, 1000)

Time (min.) (x-axis: 0, 10, 20, 30, 40, 50, 60, 70, 80, 90, 100, 110, 120)

● rsfliC LAMP with Template
○ Negative Control (No Template DNA)

FIG. 11

FIG. 12

FIG. 13

FIG. 14

FIG. 15

EP 2 585 617 B1

FIG. 16

FIG. 17

FIG. 18

EP 2 585 617 B1

FIG. 19

EP 2 585 617 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61357428 A

### Non-patent literature cited in the description

- **N. TSUGUNORI et al.** Loop-mediated isothermal amplification of DNA. *Nucleic Acids Research,* 15 June 2000, vol. 28 (12
- **Y. MORI et al.** Detection of loop-mediated isothermal amplification reaction by turbidity derived from magnesium pyrophosphate formation. *Biochemical. Biophys. Res. Comm.,* 23 November 2001, vol. 289 (1), 150-154
- **K. NAGAMINE.** Isolation of single-stranded DNA from loop-mediated isothermal amplification products. *Biochemical and Biophysical Research Communications,* 01 February 2002, vol. 290 (4), 1195-1198
- **K. NAGAMINE et al.** Accelerated reaction by loop-mediated isothermal amplification using loop primers. *Molecular and Cellular Probes,* June 2002, vol. 16 (3), 223-229
- **KUBOTA, R. ; B. G. VINE ; A. M. ALVAREZ ; D. M. JENKINS.** Detection of Ralstonia solanacearum by loop-mediated isothermal amplification. *Phytopathology,* 2008, vol. 98 (9), 1045-1051
- **NORMAN, D. ; ALVAREZ, A. M.** A rapid method for presumptive identification of Xanthomonas campestris pv. dieffenbachiae and other xanthomonads. *Plant Disease,* 1989, vol. 73, 654-658
- **YI, J. Z. ; W. D. ZHANG ; D. Y. ZHANG.** Molecular Zipper: a fluorescent probe for real-time isothermal DNA amplification. *Nucleic Acids Research,* 2006, 34